# EUROPÄISCHE PATENTSCHRIFT

(11) **EP 0 930 866 B1**
(45) Veröffentlichungstag und Bekanntmachung des Hinweises auf die Patenterteilung: **26.06.2002**
(21) Anmeldenummer: 97911168.9
(22) Anmeldetag: 02.10.1997
(51) Int. Cl.: A61K 7/00

(54) **KOSMETISCHE ODER DERMATOLOGISCHE GELE AUF DER BASIS VON MIKROEMULSIONEN**
COSMETIC OR DERMATOLOGICAL MICROEMULSION BASED GELS
GELS COSMETIQUES OU DERMATOLOGIQUES A BASE DE MICROEMULSIONS

(30) Priorität: 04.10.1996 DE 19640877; 12.10.1996 DE 19642090
(43) Veröffentlichungstag der Anmeldung: 28.07.1999
(73) Patentinhaber: Beiersdorf Aktiengesellschaft, 20245 Hamburg (DE)
(72) Erfinder: DIEC, Khiet, Hien, D-22523 Hamburg (DE); MEIER, Wolfgang, CH-4053 Basel (CH); SCHREIBER, Jörg, D-22087 Hamburg (DE)
(86) Internationale Anmeldenummer: EP9705418
(87) Internationale Veröffentlichungsnummer: WO9815254

(56) Entgegenhaltungen:
- EP-A- 0 529 883
- EP-A- 0 815 837
- WO-A-96/28132
- DE-A- 4 411 557

## Beschreibung

Die vorliegende Erfindung betrifft kosmetische oder dermatologische Gele auf der Basis von Mikroemulsionen, insbesondere solche Gele für kosmetische und dermatologische Zubereitungen. Als besondere Ausführungsform betrifft die vorliegende Erfindung kosmetische oder dermatologische Gele auf der Basis von Mikroemulsionen vom Typ Wasser in Öl, Verfahren zu ihrer Herstellung sowie ihre Verwendung für kosmetische und medizinische Zwecke.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrükkenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca 0,2 - 1,0 % eines Geliermittels) und dabei durchaus feste Konsistenz besitzen. Der Wasseranteil liegt dabei in eisähnlichen Strukturelementen vor, so daß Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind und Öl/Wasser-Gele, welche letztlich auf O/W- oder W/O-Emulsionen basieren, in welchen zusäzlich aber auch Merkmale einer Gelstruktur verwirklicht sind. Meistens sind Gele durchsichtig. In der kosmetischen bzw. pharmazeutischen Galenik zeichnen sich Gele in aller Regel durch halbfeste, oft fließfähige Konsistenz aus.

In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Die Tröpfchendurchmesser der gewöhnlichen Emulsionen liegen im Bereich von ca 1 µm bis ca. 50 µm. Solche "Makroemulsionen" sind, ohne weitere färbende Zusätze, milchigweißgefärbt und opak. Feinere "Makroemulsionen", deren Tröpfchendurchmesser im Bereich von ca. 10⁻¹ µm bis ca. 1 µm liegen, sind, wiederum ohne färbende Zusätze, bläulichweißgefärbt und undurchsichtig.

Mizellaren und molekularen Lösungen mit Partikeldurchmessern kleiner als ca. 10⁻² µm, ist vorbehalten, klar und transparent zu erscheinen.

Der Tröpfchendurchmesser von transparenten bzw. transluzenten Mikroemulsionen dagegen liegt im Bereich von etwa 10⁻² µm bis etwa 10⁻¹ µm. Solche Mikroemulsionen sind meist niedrigviskos.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten. Auch hier ist der hohe Gehalt an Emulgatoren von Nachteil.

Vorteil von Mikroemulsionsgelen ist, daß in der dispersen Phase Wirkstoffe feindispers vorliegen können. Ein weiterer Vorteil ist, daß sie aufgrund ihrer niedrigen Viskosität versprühbar sein können. Werden Mikroemulsionen als Kosmetika verwendet, zeichnen sich entsprechende Produkte durch hohe kosmetische Eleganz aus.

Es ist an sich bekannt, die Tröpfchen einer niedrigviskosen, insbesondere dünnflüssigen Mikroemulsion mit vernetzenden Substanzen miteinander zu verknüpfen, um auf diese Weise das dreidimensionale Netzwerk eines Geles zu erhalten.

In Nachr. Chem. Techn. Lab. 43 (1995) Nr. 1, S. 9 ff. werden zur Vernetzung von Mikroemulsionströpfchen kettenförmige, hydrophile Moleküle beschrieben, welche an den beiden Kettenenden je einen hydrophoben Rest aufweisen. Jene hydrophoben Reste tauchen in Mikroemulsionströpfchen ein, wobei die hydrophilen Kettenbereiche in der kontinuierlichen Wasserphase vorliegen. Im strengen Sinne ist es wohl nicht nötig, daß die hydrophoben Reste "eintauchen". Es kann im Einzelfalle auch durchaus genügen, wenn durch hydrophobe Wechselwirkung die hydrophoben Reste mit der Oberfläche der Mikroemulsionströpfchen in Kontakt treten und mehr oder weniger stark an dieser haften bleiben.

Als Vernetzer werden a.a.O. Polyoxyethylenglycole mit Oleylgruppen als hydrophobe Endgruppen angegeben.

Nachteilig an Mikroemulsionen, und somit auch an den Mikroemulsionsgelen des Standes der Technik ist, daß stets ein hoher Gehalt an einem oder mehreren Emulgatoren eingesetzt werden muß, da die geringe Tröpfchengröße eine hohe Grenzfläche zwischen den Phasen bedingt, welche in der Regel durch Emulgatoren stabilisiert werden muß.

An sich ist die Verwendung der üblichen kosmetischen Emulgatoren zwar unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, daß bestimmte Lichtdermatosen durch gewisse Emulgatoren, aber auch durch verschiedene Fette, und gleichzeitige Exposition von Sonnenlicht ausgelöst werden. Solche Lichtdermatosen werden auch "Mallorca-Akne"genannt. Eine Aufgabe der vorliegenden Erfindung war daher, Sonnenschutzprodukte zu entwickeln.

So betrifft die vorliegende Erfindung als besondere Ausführungsformen kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird im allgemeinen der engere Bereich um 308 nm angesehen.

Zum Schutze gegen UVB-Strahlung sind zahlreiche Verbindungen bekannt, bei denen es sich zumeist um Derivate des 3-Benzylidencamphers, der 4-Aminobenzoësäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Um diesen Reaktionen vorzubeugen, können den kosmetischen bzw. dermatologischen Formulierungen zusätzlich Antioxidantien und/oder Radikalfänger einverleibt werden.

UV-Absorber bzw. UV-Reflektoren sind die meisten anorganischen Pigmente, die bekannterweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen.

Mikroemulsionsgele eignen sich auch für andere kosmetische dermatologische Anwendungen, beispielsweise Desodorantien, so daß die vorliegende Erfindung in einer besonderen Ausführungsform Mikroemulsionsgele als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

Auch die Verwendung von Mikroemulsionen als Grundlage für desodorierende oder antitranspirant wirkende Zubereitungen sind bekannt. Deren relativ hoher Gehalt an Emulgatoren, mit den geschilderten Nachteilen, war bisher ein Übelstand, dem es abzuhelfen galt.

Eine weitere Aufgabe der vorliegenden Erfindung war es also, Zubereitungen zu entwickeln, welche als Grundlage für kosmetische Desodorantien bzw. Antitranspirantien geeignet sind, und die Nachteile des Standes der Technik nicht aufweisen.

Weiterhin war es eine Aufgabe der Erfindung, kosmetische Grundlagen für kosmetische Desodorantien zu entwickeln, die sich durch gute Hautverträglichkeit auszeichnen.

Ferner war eine Aufgabe der vorliegenden Erfindung, Produkte auf der Basis von Mikroemulsionsgelen mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen, aber auch ausgesprochen medizinisch-pharmazeutische Darreichungsformen geschaffen werden, zum Beispiel Zubereitungen gegen Akne und andere Hauterscheinungen.

In einer besonderen Ausführungsform betrifft die Erfindung daher Mikroemulsionen zur Befeuchtung der Haut, bzw. zur Stabilisierung von empfindlichen Wirkstoffen wie z.B. Vitamin C oder von Enzymen.

Wasserfestes Augen-Make-up, beispielsweise Mascara, ist mit Make-up-Entfernern auf wäßriger Basis nur mit speziellen Tensiden zufriedenstellend zu entfernen. Diese Tenside besitzen aber oft eine nur begrenzte physiologische Verträglichkeit. Bei einem Kontakt solcher Stoffe mit der Schleimhaut, insbesondere der Augenschleimhaut, führen diese Stoffe zu Reizungen, die sich beispielsweise in einer Rötung der Augen äußern. Reaktionen dieser Art sind typisch für tensidhaltige Produkte.

Eine Aufgabe der vorliegenden Erfindung war mithin, solchen Problemen Abhilfe zu schaffen.

Die vorliegende Erfindung betrifft in einer weiteren Ausführungsform haarkosmetische Zubereitungen. Insbesondere betrifft die vorliegende Erfindung haarkosmetische Zubereitungen zur Pflege des Haars und der Kopfhaut. In einer bevorzugten Ausführungsform betrifft die vorliegende Erfindung Zubereitungen, die dazu dienen, das einzelne Haar zu kräftigen und/oder der Haartracht insgesamt Halt und Fülle zu verleihen.

Das menschliche Haar kann, grob verallgemeinert, unterteilt werden in den lebenden Teil, die Haarwurzel, und den toten Teil, den Haarschaft. Der Haarschaft seinerseits besteht aus der Medulla, welche allerdings entwicklungsgeschichtlich bedingt für den neuzeitlichen Menschen unbedeutend geworden und zurückgebildet ist und bei dünnem Haar oft gänzlich fehlt, ferner dem die Medulla umschließenden Cortex und der die Gesamtheit aus Medulla und Cortex umhüllenden Cuticula.

Insbesondere die Cuticula, aber auch der keratinöse Bereich zwischen Cuticula und Cortex als Außenhülle des Haares sind besonderer Beanspruchung durch Umwelteinflüsse, durch Kämmen und Bürsten, aber auch durch Haarbehandlung, insbesondere Haarfärbung und Haarverformung, z.B. Dauerwellverfahren, ausgesetzt.

Bei besonders aggressiver Beanspruchung, beispielsweise der Bleichung mit Oxidantien wie Wasserstoffperoxid, bei welcher die im Cortex verteilten Pigmente oxidativ zerstört werden, kann auch das Innere des Haars in Mitleidenschaft gezogen werden. Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar. Tatsache ist, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch die Haarwäsche mit aggressiven Tensiden kann das Haar beanspruchen, zumindest dessen Erscheinungsbild oder das Erscheinungsbild der Haartracht insgesamt herabsetzen. Beispielsweise können bestimmte wasserlösliche Haarbestandteile (z.B. Harnstoff, Harnsäure, Xanthin, Keratin, Glycogen, Citronensäure, Milchsäure) durch die Haarwäsche herausgelaugt werden.

Aus diesen Gründen werden seit geraumer Zeit teils Haarpflegekosmetika verwendet, welche dazu bestimmt sind, nach Einwirken aus dem Haar wieder ausgespült zu werden, teils solche, welche auf dem Haar verbleiben sollen. Letztere können so formuliert werden, daß sie nicht nur der Pflege des einzelnen Haars dienen, sondern auch das Aussehen der Haartracht insgesamt verbessern, beispielsweise dadurch, daß sie dem Haar mehr Fülle verleihen, die Haartracht über einen längeren Zeitraum fixieren oder seine Frisierbarkeit verbessern.

Durch quaternäre Ammoniumverbindungen beispielsweise läßt sich die Kämmbarkeit der Haare entscheidend verbessern. Solche Verbindungen ziehen auf das Haar auf und sind oft noch nach mehreren Haarwäschen auf dem Haar nachweisbar.

Der Stand der Technik ließ es aber an Wirkstoffen und Zubereitungen mangeln, welche dem geschädigten Haar in befriedigender Weise Pflege zukommen ließen. Auch erwiesen sich Zubereitungen, die der Haartracht Fülle geben sollten, oft als unzureichend, zumindest waren sie ungeeignet, als Haarpflegezubereitungen eingesetzt zu werden. Die Haartracht fixierende Zubereitungen des Standes der Technik enthalten beispielsweise in der Regel viskose Bestandteile, welche Gefahr laufen, ein Gefühl der Klebrigkeit zu erwecken, welches oft durch geschickte Formulierung kompensiert werden muß.

Aufgabe war daher, auch diesen den Nachteilen des Standes der Technik Abhilfe zu schaffen.

Ferner sind Beispiele zur Herstellung transparenter Zubereitungen vom Typ Wasser in Öl mit einem niedrigem Gehalt an Emulgatoren nicht beschrieben, die auf angefeuchteter Haut weiße Makroemulsionen oder weiße Makroemulsionsgele ergeben.

Eine besondere Aufgabe der vorliegenden Erfindung war es, gelartige Zubereitungen auf Basis feindisperser Systeme vom Typ Wasser in Öl mit einem möglichst niedrigen Emulgatorgehalt zur Verfügung zu stellen, welche nicht die Nachteile des Standes der Technik aufweisen und welche für verschiedenste kosmetische und/oder dermatologische Anwendungen, beispielsweise die vorab beschreibenen Verwendungen finden können. Eine weitere Aufgabe der Erfindung war, das begrenzte Angebot an gelartigen Zubereitungen auf Basis feindisperser Systeme vom Typ Wasser in Öl des Standes der Technik zu bereichern.

Es ist an sich bekannt, W/O Mikroemulsionsgele herzustellen, In WO 92/18147 werden W/O-Mikroemulsionen beschrieben, die durch Zusatz von Wasser in O/W Mikroemulsionen überführt werden. Zur Herstellung dieser W/O Mikroemulsionsgele benötigt man allerdings eine hohe Emulgatorkonzentration.
In WO 94/22420 werden Mikroemulsionsgele beschrieben, die Silikonöle und -emulgatoren (Dimethicon Copolyol und Cyclomethicon/Dimethicon) enthalten und sich durch einen hohen Gehalt an Propylenglycol und Glycerin auszeichnen. Die Verdünnung des Gels erfolgt mit Treibgasen, sodaß nach der Applikation auf der Haut das Treibgas verdampft und ein Gel auf der Haut erzeugt wird. Dieser Weg zur Herstellung von Mikroemulsiongelen in Form eines Aerosols konnte nicht den Weg zu der Erfindung weisen.
In US 5162378 werden W/O Mikroemulsionen beschrieben, die als Emulgatoren eine Mischung von 8-20% von Cetyl Dimethicon Copolyolen, Polyglycerinestern und Hexyllaurat enthalten.
In EP 216557 A2 werden translucente W/O-Mikroemulsionenen beschrieben, wobei als Ölphase Mineralöle und als Emulgatoren Polyglycerinester verwendet werden. Die Transparenz dieser Emulsionen wird durch Angleichung der Brechungsindices von Wasser und Ölphase erreicht.
In US 5045337 werden W/O-Mikroemulsionen für den Nahrungsmittelbereich beschrieben, die sich durch sehr hohe Anteile an Lipiden auszeichnen (90-99.8%). Die verwendeten Emulgatoren auf der Basis von Poyglycerinestern können daher in ihrer Konzentration klein gehalten werden, da die Menge des solubilisierten Wassers nur 0.1 bis 5.% beträgt.

Zur Solubilisierung wasserlöslicher Wirkstoffe ist es von besonderem Vorteil, größere Mengen an Wasser in einer kosmetischen Ölphase zu micellisieren bei gleichzeitig geringem Anteil an Emulgatoren. Auffällig ist, daß die Erzeugung von Mikroemulsionsgelen für den kosmetischen oder dermatologischen Bereich mit einem niedrigen Gehalt an Emulgatoren, höherer Konzentration an solubilisiertem Wasser, breiter Variationsmöglichkeit der Ölkomponenten und die Vernetzung von Wassertropfen durch hydrophil modifizierte lipophile Polymere nicht beschrieben ist.

All diesen Übelständen galt es also, abzuhelfen.

Erstaunlicherweise werden alle der Erfindung zugrundliegenden Aufgaben gelöst durch Mikroemulsionen oder Mikroemulsionsgele vom Typ Wasser in Öl mit einem niedrigem Gehalt an Emulgatoren, die auf angefeuchteter Haut (Duschen, Waschen des Gesichts, Körpers) weiße Makroemulsionen oder weiße Makroemulsionsgele ergeben.

Erstaunlicherweise werden alle der Erfindung zugrundliegenden Aufgaben gelöst durch Mikroemulsionsgele,
(a) beruhend auf Mikroemulsionen vom Typ Wasser in Öl, welche umfassen
   - eine Ölphase, welche im wesentlichen aus schwerflüchtigen Bestandteilen zusammengesetzt ist, und eine Wasserphase
   - enthaltend:
      einen oder mehrere polyethoxylierte W/O-Emulgatoren und/oder
      einen oder mehrere polypropoxylierte W/O-Emulgatoren und/oder
      einen oder mehrere polyethoxylierte und polypropoxylierte W/O-Emulgatoren und/oder
   - einen oder mehrere Monoester, Diester, Polyester von Polyolen als W/O-Emulgatoren und/oder
   - einen oder mehrere Monoether, Diether, Polyether von Polyolen als W/O-Emulgatoren und/oder
   - einen oder mehrere Dimethicon copolyole als W/O Emulgatoren und/oder
   - einen oder mehrere Fettalkohole oder Fettsäuren als W/O-Emulgatoren und/oder
   - einen oder mehrere Sorbitanester als W/O-Emulgatoren und/oder
   - einen oder mehrere Methylglucoseester als W/O-Emulgatoren
   - gewünschtenfalls ferner enthaltend einen oder mehrere O/W-Emulgatoren
   - erhältlich auf die Weise, daß ein Gemisch aus den Grundkomponenten, umfassend Wasserphase, Ölphase, einen oder mehrere der erfindungsgemäßen Emulgatoren hergestellt wird, wobei der HLB-Wert des Emulgators oder der Emulgatorkombination im Bereich von 2 - 14 liegt,
(b) bei welcher die Tröpfchen der diskontinuierlichen Wasserphase durch eine oder mehrere Vernetzersubstanzen miteinander verbunden sind, deren Moleküle sich durch mindestens einen hydrophoben Bereich auszeichnen, welcher eine Ausdehnung aufweist, die geeignet ist, den Abstand der Mikroemulsionströpfchen untereinander zu überbrücken, und durch mindestens einen hydrophilen Bereich, welcher mit den Mikroemulsionströpfchen in hydrophile Wechselwirkung zu treten vermag.

Vorzugsweise liegen die Gewichtsprozente der Emulgatoren < 20%, ganz besonders bevorzugt 0,1 - 10% bezogen auf das Gesamtgewicht der Mikroemulsion.

Vorzugsweise enthalten also die erfindungsgemäßen Mikroemulsionsgele mindestens einen der unter (a) genannten W/O-Emulgatoren, gegebenenfalls aber auch ein Gemisch von mehreren solchen W/O-Emulgatoren. Der HLB-Wert des Emulgators oder der Emulgatoren-Kombination kann auch im Bereich von 2 - 7 liegen.

Es ist dabei gleichermaßen vorteilhaft, wenn die Vernetzersubstanz, im Rahmen der vorliegenden Beschreibung auch als Verdicker bezeichnet, ein eigenständiges Gelnetzwerk bildet, in welchem die Mikroemulsionströpfchen dann durch hydrophile Wechselwirkung festgehalten werden (dann liegen sogenannte assoziative Verdicker vor), oder ob der Zusammenhalt des Netz durch die Vernetzung mit den Mikroemulsionströpfchen in den Knotenpunkten des Netzes bewirkt wird. Ferner können die Endgruppen des hydrophoben Polymers auch ionischen Charakter haben und z.B. Carboxylate oder Sulfonate sein.

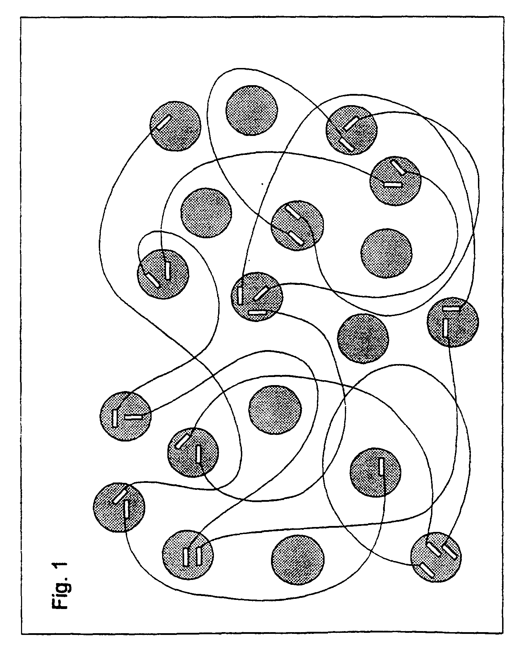

In Fig. 1 wird dieses erfindungsgemäße Prinzip für W/O-Mikroemulsionen verdeutlicht: Die als schraffierte Kreise dargestellt Mikroemulsionströpfchen einer W/O-Mikroemulsion werden durch die als Linien dargestellten Vernetzermoleküle miteinander verbunden, welche an beiden Enden durch Rechtecke symbolisierte hydrophile Reste tragen. Ersichtlich ist, daß ein Wassertröpfchen grundsätzlich auch mehrere hydrophile Reste beherbergen kann, wodurch eine stärkere Vernetzung und Dreidimensionalität des Netzwerks gewährleistbar ist.

Die erfindungsgemäß verwendeten Vernetzersubstanzen folgen in der Regel Strukturschemata wie folgt: 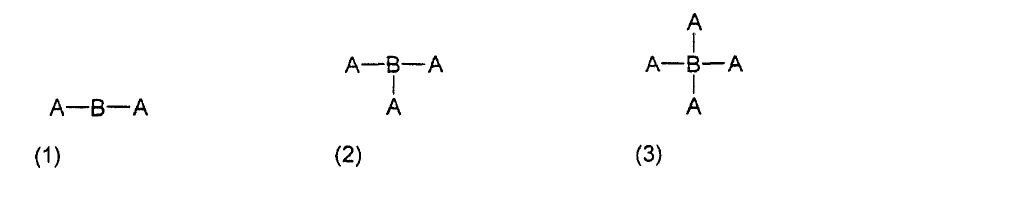 wobei B einen hydrophoben Bereich des jeweiligen Vernetzermoleküls symbolisieren kann und A jeweils hydrophile Bereiche, welche auch innerhalb eines Moleküls unterschiedlicher chemischer Natur sein mögen.

Aber auch Strukturschemata wie 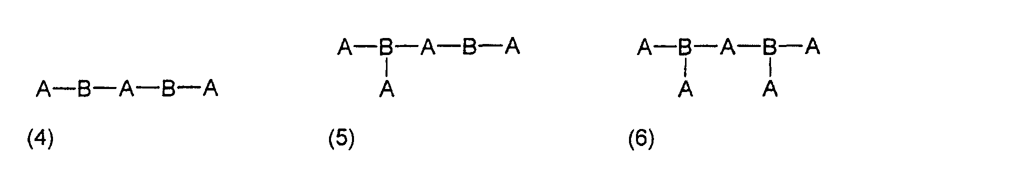 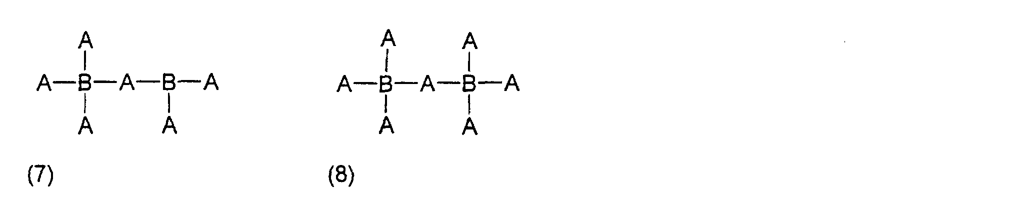 und analog gebildete, noch komplexere Strukturen fallen durchaus in den Rahmen der hiermit vorgelegten Erfindung.

Ebenfalls in den Rahmen der hiermit vorgelegten Erfindung fallen Strukturschemata wie folgt: 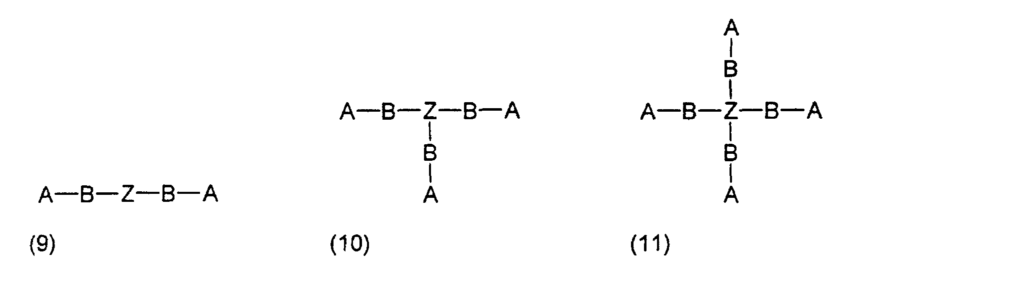 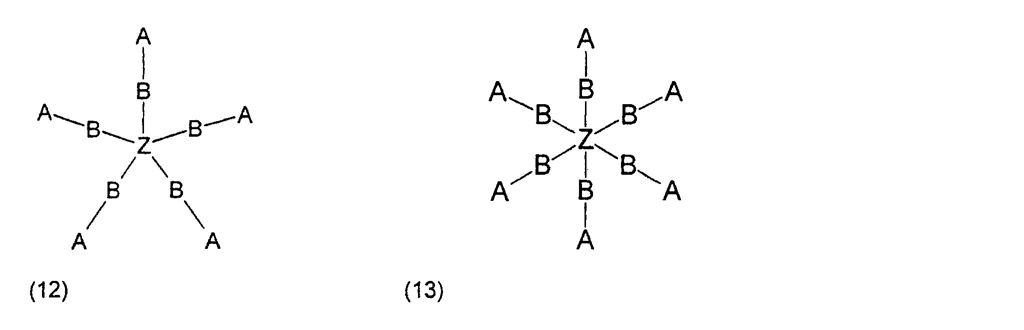 wobei Z dabei eine Zentraleinheit darstellt, welche hydrophil oder hydrophob sein kann und in der Regel aus einem oligo- oder polyfunktionellen Molekülrest besteht.

Selbstverständlich fallen auch Verdicker mit höherem Verzweigungsgrad in den Rahmen der vorliegenden Erfindung.

Beispielsweise kann Z in Schema (10) aus einem Glycerylrest bestehen, dessen drei OH-Funktionen in die Bereiche B übergehen, welche ihrerseits beispielsweise aus (SiOMe₂)-Ketten gleicher oder ungleicher Länge bestehen, und deren terminale Gruppe z.B. mit einer ethoxylierten Gruppe kovalent verknüpft ist. Zwischen der funktionellen Gruppe (hier O - Atom) und der hydrophoben Gruppe (hier SiOMe₂) können auch weitere Gruppen (Alkyl, Aryl, Hetaryl, gesättigt, ungesättigt) insertiert sein.
Auch Teilsubstitution an Glycerin ist möglich, wodurch Strukturen entstehen, welche Schema (9) entsprechen.

Vorteilhaft können die hydrophoben Gruppen B so gewählt werden, daß der Vernetzer insgesamt öllöslich oder zumindest in Öl dispergierbar ist, wobei dann der hydrophile Anteil der Gruppen A überkompensiert werden sollte.

Für das Strukturschema (1) können beispielsweise folgende spezielleren Strukturschemata befolgt werden: 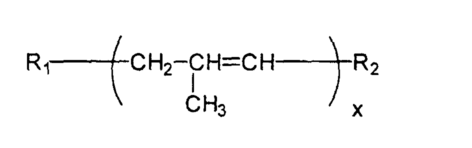 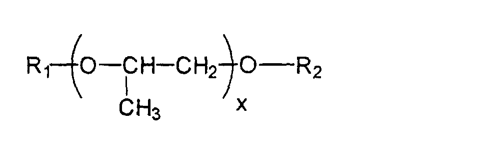 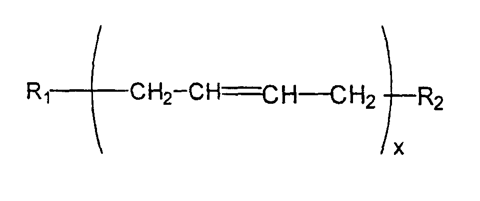 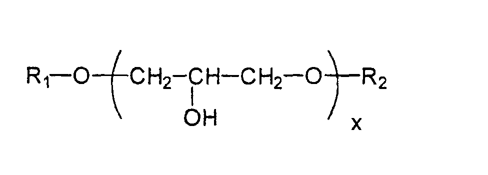 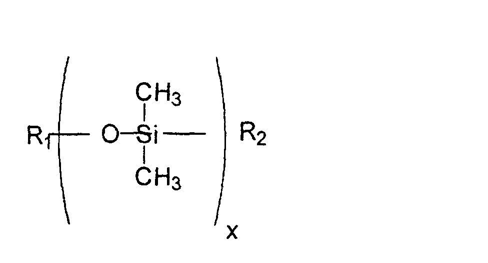 wobei R₁, R₂, unabhängig voneinander verzweigte oder unverzweigte, gesättigte oder ungesättigte, cyclische oder kettenförmige aliphatische, aromatische oder heteroarmatische Reste darstellen können, die den wasserlöslichen/dispergierbaren Teil des Polymers bilden. Diese Reste können z. B Polyole sein (z.B. Sorbitol, Glycerin, Pentaerythrit, Sucrose, Polyglycerin), Glucane, Zucker, Carboylat-Gruppen, Sulfonatgruppen, Sulfat-Gruppen, mit Hydroxygruppen und/oder Sulfonsäure-Gruppen und/oder Carboxylat-Gruppen und/oder Sulfatgruppen substituierte Aromaten bzw. Heteroarmaten bzw. SiR(OH)ₙ-Gruppen.
x bedeutet dabei eine Zahl, die es dem Gesamtmolekül erlaubt, in Öl löslich oder zumindest dispergierbar zu sein, typischerweise gewählt aus dem Bereich größer als 10, vorteilhaft aus dem Bereich 20 - 5000. Im Einzelfalle kann die Zahl x auch noch wesentlich höhere Werte, sogar mehrere Millionen, annehmen. Dies ist dem Fachmanne an sich bekannt und bedarf keiner weiteren Erläuterung.

Für das Strukturschema (10) können beispielsweise folgende spezielleren Strukturschemata befolgt werden: 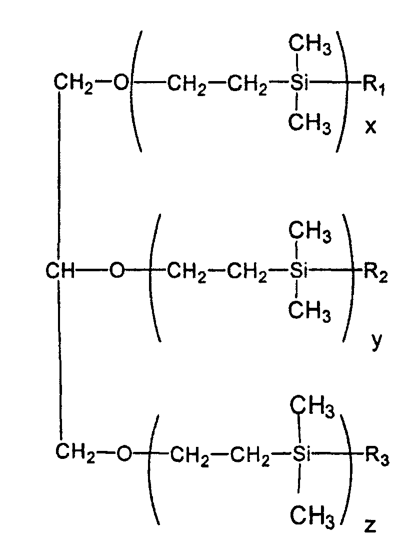 wobei R₁, R₂, R₃ unabhängig voneinander verzweigte oder unverzweigte, gesättigte oder ungesättigte, cyclische oder kettenförmige aliphatische, aromatische oder heteroaromatische Reste darstellen können, die den wasserlöslichen/dispergierbaren Teil des Polymers bilden. Diese Reste können z. B Polyole sein (Glycerin, Pentaerythrit, Sucrose, Polyglycerin), Glucane, Zucker, Carboylat-Gruppen, Sulfonatgruppen, Sulfat-Gruppen, mit Hydroxygruppen und/oder Sulfonsäure-Gruppen und/oder Carboxylat-Gruppen und/oder Sulfatgruppen substituierte Aromaten bzw. Heteroarmaten bzw. SiR(OH)ₙ-Gruppen. x, y , z bedeuten dabei Zahlen, die es dem Gesamtmolekül erlaubt, in Öl löslich oder zumindest dispergierbar zu sein, typischerweise gewählt aus dem Bereich größer als 10, vorteilhaft aus dem Bereich 20 - 5000.
Auch Teilsubstitution ist dabei möglich, wobei einer oder mehrere der Indices x, y, z den Wert Null annehmen können und einer oder mehrere der Reste R₁, R₂ oder R₃ Wasserstoffatome darstellen können.

Für das Strukturschema (11) können beispielsweise folgende spezielleren Strukturschemata befolgt werden: 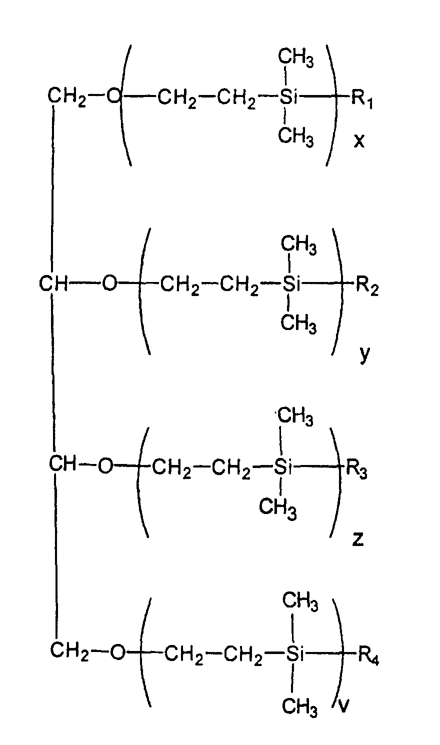 wobei R₁, R₂, R₃, R₄ unabhängig voneinander verzweigte oder unverzweigte, gesättigte oder ungesättigte, cyclische oder kettenförmige aliphatische oder aromatische Reste darstellen können, die den wasserlöslichen/dispergierbaren Teil des Polymers bilden. Diese Reste können z. B Polyole sein (Glycerin, Pentaerythrit, Sucrose, Polyglycerin), Glucane, Zucker, Carboxylat-Gruppen, Sulfonatgruppen, Sulfat-Gruppen, mit Hydroxygruppen und/oder Sulfonsäure-Gruppen und/oder Carboxylat-Gruppen und/oder Sulfatgruppen substituierte Aromaten bzw. Heteroarmaten bzw. SiR(OH)ₙ-Gruppen. x, y, v und z bedeuten dabei Zahlen, die es dem Gesamtmolekül erlaubt, in Öl löslich oder zumindest dispergierbar zu sein, typischerweise gewählt aus dem Bereich größer als 10, vorteilhaft aus dem Bereich 20 - 5000.
Auch hier gilt selbstverständlich, daß Teilsubstitution möglich ist, wobei einer oder mehrere der Indices x, y, z und v den Wert Null annehmen können und einer oder mehrere der Reste R₁, R₂ ,R₃ oder R₄ Wasserstoffatome darstellen können. Dabei gehen die Substanzen natürlich in andere Strukturschemata über.

Es ist auch von Vorteil, Verdicker zu wählen, die an den Polymerenden Verzweigungen aufweisen, sodaß Dendrimere erhalten werden.

Als besonders geeignete Vernetzer haben sich solche herausgestellt, gewählt aus der Gruppe der
- hydrophil substituierten Polyisoprene
- der Dimethicon Copolyole der allgemeinen Formel
   R-Si(CH₃)₂O-(Si(CH₃)₂O)ₙ - Si(CH₃)₂R', wobei R und R' unabhängig voneinander verzweigte oder unverzweigte, gesättigte oder ungesättigte, cyclische oder kettenförmige aliphatische, aromatische oder heteroaromatische hydrophobe Gruppen darstellen können, an die hydrophile Gruppen kovalent gebunden sind, so daß insgesamt die Reste R und R' wasserlöslich oder dispergierbar sind, die die im folgenden die für hydrophile Gruppen genannten Strukturen haben können. Es ist auch möglich, für R und R' ausschließlich hydrophile Gruppen zu wählen ohne hydrophobe Teilstrukturen, wobei dann R und R' unabhängig voneinander verzweigte oder unverzweigte, gesättigte oder ungesättigte, cyclische oder kettenförmige aliphatische, aromatische, heteroaromatische Gruppen darstellen.

Bevorzugte Dimethicon Copolyole haben die allgemeine Formel (A)

R-Si(CH₃)₂O- (Si(CH₃)₂O)ₙ - Si(CH₃)₂R',

wobei n so gewählt wird, daß das gesamte Molekül fettlöslich/dispergierbar ist und vorzugsweise im Bereich von 30 - 10⁷ gewählt wird und die Reste R und R' unabhängig voneinander die folgenden Strukturelemente bedeuten:

R, R' = (CH₂)_{y}-(O-CH₂-C(OH)H-CH₂)ₓOH

R, R' = (CH₂)_{y}-(OC₂H₄)ₓ-OH

wobei y z.B. Werte von 0 - 10⁶ bedeuten kann, bevorzugt 1 - 30, insbesondere 2 bis 20, und x z.B. Werte von 1 bis 10⁷ bedeuten kann, bevorzugt 2 bis 10⁶, insbesondere 3 bis 500,

Besonders bevorzugt werden Dimethicon Copolyole der vorstehenden Formel A, worin R, R' = CH₂CH₂CH₂(OCH₂CH₂)₁₂OH ist und n = 148 bedeutet und im folgenden A1 genannt wird.

Bevorzugte hydrophil substituierte Polyisoprene besitzen z.B. zwei Ethylenoxidendgruppen oder Sulfatendgruppen oder Carboxylatendgruppen.

Es kann auch gegebenenfalls vorteilhaft sein, wenn der oder die erfindungsgemäß verwendeten Verdicker über physiologische Wirksamkeit im Sinne einer kosmetischen oder pharmazeutischen Wirkung verfügt oder verfügen. So können beispielsweise die hydrophilen Endgruppen des Polymers das Gerüst der Alginate tragen.

Die Menge der erfindungsgemäßen Verdicker sollte vorzugsweise im Bereich von 0,3 bis 30 Gew.-%, insbesondere 1 bis 10 Gew.-% liegen, jeweils bezogen auf das Gesamtgewicht der Mikroemulsion.

Die Praxis der Herstellung eines erfindungsgemäßen Mikroemulsionsgeles besteht demgemäß vorteilhaft darin, nach Auswahl geeigneter Rohstoffe, d.h., Wasser- und Ölphase, ein oder mehrere erfindungsgemäß verwendete W/O-Emulgatoren, ein oder mehrere erfindungsgemäß verwendete Verdicker und gegebenenfalls weitere Substanzen, die Einzelkomponenten unter Rühren zusammenzugeben und durch Abkühlung des Gemisches besagte Gele zu erhalten. Dabei können während der Abkühlung flüssig kristalline Bereiche oder andere dem Fachmann geläufige Phasen durchlaufen werden.
Ferner kann auch der Verdicker nachträglich zu einer bei Raumtemperatur oder durch Abkühlung erhaltenen niedrigviskosen W/O Mikroemulsion zugesetzt werden.

Dieses erfindungsgemäße Verfahren ist besonders gut geeignet, wenn den erfindungsgemäßen W/O-Mikroemulsionsgelen wärmeempfindliche oder leichtflüchtige Substanzen einverleibt werden sollen. Darüberhinaus ist dieses bei relativ niedrigen Temperaturen durchzuführende Verfahren gegenüber üblichen Verfahren energiesparend.

Insbesondere vorteilhaft im Sinne der vorliegenden Erfindung sind Mikroemulsionsgele,
(a) beruhend auf Mikroemulsionen vom Typ Wasser-in-Öl, welche umfassen
   - eine kontiniuerliche Ölphase und eine diskontinuierliche Wasserphase
   - enthaltend mindestens einen
      einen oder mehrere polyethoxylierte W/O-Emulgatoren und/oder
      einen oder mehrere polypropoxylierte W/O-Emulgatoren und/oder
      einen oder mehrere polyethoxylierte und polypropoxylierte W/O-Emulgatoren und/oder
      einen oder mehrere Monoester, Diester, Polyester von Polyolen als W/O-Emulgatoren und/oder
   - einen oder mehrere Monoether von Polyolen und deren Ester als W/O-Emulgatoren und/oder
   - einen oder mehrere Sorbitanester als W/O-Emulgatoren und/oder
   - einen oder mehrere Silikonemulgatoren als W/O Emulgatoren und/oder
   - einen oder mehrere Fettalkohole oder Fettsäuren als W/O-Emulgatoren und/oder
   - einen oder mehrere Methylglucoseester als W/O-Emulgatoren,
   - wobei dieser W/O-Emulgator ausgewählt wird aus der Gruppe der
   - der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 1 bis 10 darstellen
   - der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 1 bis 30 darstellen
   - der Fettsäureethoxylate der allgemeinen Formel R-COO-(-CH₂-CH₂-O-)ₙ -H, wobei R einen verzweigte oder unverzweigten Alkyl- oder Alkenylreste und n eine Zahl von 1 bis 20 darstellen,
   - der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste und n eine Zahl von 1 bis 20 darstellen,
   - der veresterten Fettsäureethoxylate der allgemeinen Formel R-COO-(-CH₂-CH₂-O-)ₙ-C(O)-R', wobei R und R' unabhängig voneinander verzweigte oder unverzweigte Alkyl, Hydroxyalkyl - oder Alkenylreste und n eine Zahl von 1 bis 40 darstellen,
   - der veretherten Fettsäureethoxylate der allgemeinen Formel R-COO-(-CH₂-CH₂-O-)ₙ-R', wobei R und R' unabhängig voneinander verzweigte oder unverzweigte Alkyl- oder Alkenylreste und n eine Zahl von 1 bis 40 darstellen
   - der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 1 bis 30 darstellen,
   - der Polyoxyethylensorbitanfettsäureester, basierend auf verzweigten oder unverzweigten Alkan- oder Alkensäuren und einen Ethoxylierungsgrad von 1 bis 10 aufweisend
   - der Cholesterinethoxylate mit einem Ethoxylierungsgrad zwischen 1 und 10,
   - der ethoxlierten Glyceride mit einem Ethoxylierungsgrad von 1 bis 30
   - der ethoxylierten Triglyceride mit einem Ethoxylierungsgrad zwischen 1 und 30,
   - der Monoglycerinether des Typs R-O-CH₂-C(H)OH-CH₂OH wobei R einen verzweigten oder unverzweigten Alkyl-, Aryl- oder Alkenylrest darstellen und
   - der Monoglycerinester des Typs RC(O)OCH₂-C(H)OH-CH₂OH wobei R einen verzweigten oder unverzweigten Alkyl-, Hydroxyalkyl, Aryl- oder Alkenylrest darstellen
   - der Diglycerinester des Typs RC(O)OCH₂-C(H)OH-CH₂OC(O)R', wobei wobei R und R' unabhängig voneinander verzweigte oder unverzweigte Alkyl- , Hydroxyalkyl oder Alkenylreste und n eine Zahl von 1 bis 30 oder darstellen,
   - der Polyglycerinmono- oder di- oder polyester, wobei die Fettsäuren unabhängig voneinander verzweigte oder unverzweigte Alkyl- , Hydroxyalkyl oder Alkenylreste darstellen,
   - der Pentaerythritester wobei die Fettsäuren unabhängig voneinander verzweigte oder unverzweigte Alkyl-, Hydroxyalkyl oder Alkenylreste darstellen,
   - der Propylenglycolester, wobei die Fettsäuren unabhängig voneinander verzweigte oder unverzweigte Alkyl- , Hydroxyalkyl oder Alkenylreste darstellen,
   - der Sorbitanester, wobei die Fettsäuren unabhängig voneinander verzweigte oder unverzweigte Alkyl- , Hydroxyalkyl oder Alkenylreste darstellen,
   - der Fettalkohole R-OH und Fettsäuren RCOOH, wobei R einen verzweigten oder unverzweigten Alkyl- oder Alkenylrest darstellen,
   - der Silikonemulgatoren
   - der Methylglucoseester, wobei die Fettsäuren unabhängig voneinander verzweigte oder unverzweigte Alkyl- , Hydroxyalkyl oder Alkenylreste darstellen
   - gewünschtenfalls enthaltend einen oder mehrere O/W-Emulgatoren
   - einen Gesamtemulgatorgehalt vorzugsweise kleiner als 20 Gew.-%, bezogen auf das Gesamtgewicht der Emulsion, aufweisend,
   - erhältlich auf die Weise, daß ein Gemisch aus den Grundkomponenten, umfassend Wasserphase, Ölphase, einen oder mehrere der erfindungsgemäßen W/O-Emulgatoren, gewünschtenfalls einen oder mehrere O/W-Emulgatoren hergestellt wird, wobei der HLB-Wert des Emulgators oder der Emulgatorkombination im Bereich von 2 - 14 liegt,
(b) bei welcher die Tröpfchen der diskontinuierlichen Wasserphase durch eine oder mehrere Vernetzersubstanzen miteinander verbunden sind, deren Moleküle sich durch mindestens einen hydrophoben Bereich auszeichnen, welcher eine Ausdehnung aufweist, die geeignet ist, den Abstand der Mikroemulsionströpfchen untereinander zu überbrücken, und durch mindestens einen hydrophilen Bereich, welcher mit den Mikroemulsionströpfchen in hydrophile Wechselwirkung zu treten vermag.

Erfindungsgemäß besonders vorteilhaft wird der Emulgator/die Emulgatormischung in einem HLB-Bereich von 2-14 eingesetzt.

Es ist von Vorteil aus dem Bereich der Monoglycerinester des Typs RC(O)OCH₂-C(H)OH-CH₂OH das Glycerinisostearat oder das Glycerinmonocaprylat einzusetzen

Es ist von Vorteil aus dem Bereich der Polyglycerinmono- oder di- oder polyester das Polyglycerindiisostearat (HLB = 6.0) und das Polyglycerinltriisostearat (HLB = 4) einzusetzen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(2)stearylether (Steareth-2).

Es ist ferner von Vorteil, die Fettsäureethoxylate aus folgender Gruppe zu wählen:
Polyethylenglycol(4)stearat.

Als veresterte Fettsäureethoxylate können vorteilhaft gewählt werden
Polyethylenglycol(12) dilaurat, Polyethylenglycol(8) distearat.

Es ist ebenfalls günstig, die ethoxylierten Sorbitanester aus der Gruppe Polyethylenglycol(20)sorbitantrioleat, Polyethylenglycol(20)sorbitantrisostearat zu wählen.

Als ethoxyliertes Cholesterinderivat kann vorteilhaft Polyethylenglycol(5) Sojasterol verwendet werden.

Als ethoxylierte Triglyceride kann vorteilhaft Polyethylenglycol(20) Glyceryltristearat verwendet werden.

Weiterhin ist von Vorteil, die Polyethylenglycolglycerinfettsäureester aus der Gruppe Polyethylenglycol(5)glycerylstearat zu wählen.

Als Monoglycerineether kann man vorteilhaft 2-Ethylhexylglycerinether verwenden.

Es ist ebenfalls günstig Diglycerinester aus der Gruppe der Polyglyceryl-2 Dipolyhydroxyfettsäuren zu wählen.

Als Sorbitanester hat sich Sorbitanmonolaurat und Sorbitanmonooleat bewährt.

Als fakultative, dennoch erfindungsgemäß vorteilhafte O/W-Emulgatoren können eingesetzt werden: höher ethoxlierte Fettalkohole mit 8 bis 30 Kohlenstoffatomen,
höher ethoxlierte Diglycerinester gesättigter und/oder ungesättigter, verzweigter und/oder unverzweigter Alkancarbonsäuren einer Kettenlänge von 8 bis 24, insbesondere 12 - 18 C-Atomen,
höher ethoxlierte Sorbitanester gesättigter und/oder ungesättigter, verzweigter und/oder unverzweigter Alkancarbonsäuren einer Kettenlänge von 8 bis 24, insbesondere 12 - 18 C-Atomen.
höher ethoxylierte veresterte Fettsäurethoxylate gesättigter und/oder ungesättigter, verzweigter und/oder unverzweigter Alkancarbonsäuren einer Kettenlänge von 8 bis 24, insbesondere 12 - 18 C-Atomen.
höher ethoxylierte Polyethylenglycolglycerinfettsäuren gesättigter und/oder ungesättigter, verzweigter und/oder unverzweigter Alkancarbonsäuren einer Kettenlänge von 8 bis 24, insbesondere 12 - 18 C-Atomen.
höher ethoxlierte Triglyceride gesättigter und/oder ungesättigter, verzweigter und/oder unverzweigter Alkancarbonsäuren einer Kettenlänge von 8 bis 24, insbesondere 12 - 18 C-Atomen.
Ferner Polyglycerinmono- und diester gesättigter und/oder ungesättigter, verzweigter und/oder unverzweigter Alkancarbonsäuren einer Kettenlänge von 8 bis 24, insbesondere 12 - 18 C-Atomen bzw. Polyglycerinester oder -ether von Methylglucoseestern.
Ferner können auch anionische oder kationische O/W-Emulgatoren eingesetzt werden wie zum Beispiel die Satze der Alkylethercarbonsäuren, Acyl Lactylate, Acyl Glutamate und Acyl Sarcosinate.

Es ist von Vorteil als kationische Emulgatoren das Cetyltrimethylamoniumbromid einzusetzen.

Es ist von Vorteil als anionische Emulgatoren das Natriumlauroyl Lactylat, das Natriumcaproyl Lactylat oder das Natriumlaurethsulfat einzusetzen.

Es ist von Vorteil aus dem Bereich der Polyglycerinmono- oder di- oder polyester das Polyglycerinmonoisostearat (HLB = 14) einzusetzen.

Die Ölphase der erfindungsgemäßen Mikroemulsionsgele wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Es kann auch gegebenenfalls vorteilhaft sein,Wachse zur Ölphase hinzuzufügen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Es ist erfindungsgemäß möglich, den Gesamtgehalt an Emulgatoren kleiner als 15 Gew.-%, bezogen auf das Gesamtgewicht der erfindungsgemäßen Mikroemulsionsgele, zu halten. Es wird bevorzugt, den Gesamtgehalt an Emulgatoren kleiner als 10 Gew.%, insbesondere kleiner als 8 Gew.-%, bezogen auf das Gesamtgewicht der Mikroemulsionsgele, zu halten.
Es ist dabei im Einzelfalle möglich, daß die vorgenannten Konzentrationsgrenzen leicht über- oder unterschritten werden und dennoch die betreffenden Emulsionstypen erhalten werden. Dies kommt angesichts der breit streuenden Vielfalt an geeigneten Emulgatoren und Ölbestandteilen für den Fachmann nicht unerwartet, so daß er weiß, daß bei solchen Über- oder Unterschreitungen der Boden der vorliegenden Erfindung nicht verlassen wird.

Die erfindungsgemäßen Mikroemulsionsgele enthalten vorteilhaft Elektrolyte, insbesondere eines oder mehrere Salze mit folgenden Anionen: Chloride, ferner anorganische Oxo-Element-Anionen, von diesen insbesondere Sulfate, Carbonate, Phosphate, Borate und Aluminate. Auch auf organischen Anionen basierende Elektrolyte können vorteilhaft verwendet werden, beispielsweise Lactate, Acetate, Benzoate, Propionate, Tartrate, Citrate und andere mehr. Vergleichbare Effekte sind auch durch Ethylendiamintetraessigsäure und deren Salze zu erzielen.

Als Kationen der Salze werden bevorzugt Ammonium,- Alkylammonium,- Alkalimetall-, Erdalkalimetall,- Magnesium-, Eisen- bzw. Zinkionen verwendet. Es bedarf an sich keiner Erwähnung, daß in Kosmetika nur physiologisch unbedenkliche Elektrolyte verwendet werden sollten. Spezielle medizinische Anwendungen der erfindungsgemäßen Mikroemulsionen können andererseits, wenigstens grundsätzlich, die Verwendung von Elektrolyten bedingen, welche nicht ohne ärztliche Aufsicht verwendet werden sollten.

Besonders bevorzugt sind Kaliumchlorid, Kochsalz, Magnesiumsulfat, Zinksulfat und Mischungen daraus. Ebenfalls vorteilhaft sind Salzmischungen wie sie im natürlichen Salz vom Toten Meer auftreten.

Die Konzentration des oder der Elektrolyte sollte etwa 0,1 - 10,0 Gew.-%, besonders vorteilhaft etwa 0,3 - 8,0 Gew.% betragen, bezogen auf das Gesamtgewicht der Zubereitung.

Die erfindungsgemäßen Mikroemulsionsgele tragen ferner in vorzüglicher Weise zur Hautglättung bei, insbesondere, wenn sie mit einer oder mehreren Substanzen versehen sind, die die Hautglättung fördern.

Stellen die erfindungsgemäßen Mikroemulsionsgele Grundlagen für kosmetische Desodorantien/Antitranspirantien dar, so können alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Mikroemulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11.Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Die üblichen Antitranspiranswirkstoffe können ebenfalls vorteilhaft in den erfindungsgemäßen Mikroemulsionsgele verwendet werden, insbesondere Adstringentien, beispielsweise basische Aluminiumchloride.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren Mikroemulsionsgelen.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z.B. in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist auch die Kombination eines erfindungsgemäßen UVA-Filters mit einem UVB-Filter bzw. eine erfindungsgemäßes kosmetische oder dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Erfindungsgemäße kosmetische und/oder dermatologische Zubereitungen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Eine erstaunliche Eigenschaft der vorliegenden Erfindung ist, daß erfindungsgemäße Zubereitungen sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrunde steht wie die Bekämpfung der oxidativen Beanspruchung der Haut. Es ist aber auch günstig, die erfindungsgemäßen Mikroemulsionsgele mit einem Gehalt an einem oder mehreren Antioxidantien zu versehen, wenn die Zubereitungen einem anderen Zwecke dienen sollen, z.B. als Desodorantien oder Sonnenschutzmittel.

Besonders vorteilhaft werden die Antioxidantien gewählt aus der Gruppe bestehend aus
Aminosäuren (z.B. Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, gamma-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptahioninsulfoximin) in sehr geringen verträglichen Dosierungen (z.B. pmol bis µmol/kg), ferner (Metall)-Chelatoren (z.B. α-Hydroxyfettsäuren, α-Hydroxypalmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. gamma-Linolensäure, Linolsäure, Ölsäure), Folsäure und deren Derivate, Ubichinon und Ubichinol deren Derivate, Vitamin C und Derivate (z.B. Ascorbylpalmitate, Mg - Ascorbylphosphate, Ascorbylacetate), Tocopherole und Derivate (z.B. Vitamin E - acetat), Vitamin A und Derivate (Vitamin A - palmitat) sowie Koniferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist dem Fachmanne natürlich bekannt, daß anspruchsvolle kosmetische Zubereitungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Erfindungsgemäß können Wirkstoffe auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z.B. Hydrocortison-17-valerat, Vitamine, z.B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die γ-Linolensäure, Ölsäure, Eicosapentaënsäure, Docosahexaënsäure und deren Derivate, Chloramphenicol, Coffein, Prostaglandine, Thymol, Campher, Extrakte oder andere Produkte pflanzlicher und tierischer Herkunft, z.B. Nachtkerzenöl, Borretschöl oder Johannisbeerkernöl, Fischöle, Lebertran aber auch Ceramide und ceramidähnliche Verbindungen und so weiter.

Obgleich selbverständlich auch die Verwendung hydrophiler Wirkstoffe erfindungsgemäß begünstigt ist, ist ein weiterer Vorteil der erfindungsgemäßen Mikroemulsionsgele, daß die hohe Anzahl feinstzerteilter Tröpfchen gerade öllösliche bzw. lipophile Wirkstoffe mit besonders großer Wirksamkeit biologisch verfügbar macht.

Vorteilhaft ist es auch, die Wirkstoffe aus der Gruppe der rückfettenden Substanzen zu wählen, beispielsweise Purcellinöl, Eucerit® und Neocerit®.

Es ist auch möglich und gegebenenfalls vorteilhaft, den erfindungsgemäßen Zubereitungen waschaktive Tenside zuzufügen. Erfindungsgemäße wäßrige kosmetische Reinigungsmittel oder für die wäßrige Reinigung bestimmte wasserarme oder wasserfreie Reinigungsmittelkonzentrate können kationische, anionische, nichtionische und/oder amphotere Tenside enthalten, beispielsweiseherkömmliche Seifen, z.B. Fettsäuresalze des Natriums, Alkylsulfate, Alkylethersulfate, Alkan- und Alkylbenzolsulfonate, Sulfoacetate, Sulfobetaine, Sarcosinate, Amidosulfobetaïne, Sulfosuccinate, Sulfobernsteinsäurehalbester, Alkylethercarboxylate, Eiweiß-Fettsäure-Kondensate, Alkylbetaïne und Amidobetaïne, Fettsäurealkanolamide, Polyglycolether-Derivate.

Kosmetische Zubereitungen, die kosmetische Reinigungszubereitungen für die Haut darstellen, können in flüssiger oder fester Form vorliegen. Sie enthalten vorzugsweise mindestens eine anionische, nicht-ionische oder amphotere oberflächenaktive Substanz oder Gemische daraus, mindestens einen erfindungsgemäßes Elektrolyten und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, gegebenenfalls Elektrolyte und Hilfsmittel, wie sie üblicherweise dafür verwendet werden. Die oberflächenaktive Substanz kann bevorzugt in einer Konzentration zwischen 1 und 50 Gew.-% in den Reinigungszubereitungen vorliegen, bezogen auf das Gesamtgewicht der Zubereitungen. Vorteilhaft sind beispielsweise Cetyltrimethylammoniumsalze zu verwenden.

Die erfindungsgemäßen für die Reinigung des Haares oder der Haut vorgesehenen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die erfindungsgemäßen Zubereitungen haben, trotz ihres Ölgehaltes, in erstaunlicher Weise hohe Reinigungskraft und wirken in hohem Maße regenerierend bzw. befeuchtend in bezug auf den allgemeinen Hautzustand. Insbesondere wirken die erfindungsgemäßen Zubereitungen hautglättend, vermindern das Trockenheitsgefühl der Haut und machen die Haut geschmeidig.

Sollen die erfindungsgemäßen Mikroemulsionsgele zur Haarpflege eingesetzt werden, können sie die üblichen Bestandteile enthalten, üblicherweise zum Beispiel filmbildende Polymere. Von solchen Polymeren mit wenigstens teilweise quaternisierten Stickstoffgruppen (im folgenden "Filmbildner" genannt), eigenen sich bevorzugt solche, welche gewählt werden aus der Gruppe der Substanzen, welche nach der INCI-Nomenklatur (International Nomenclature Cosmetic Ingredient) den Namen "Polyquaternium" tragen, beispielsweise:
- Polyquaternium-2: (Chemical Abstracts-Nr. 63451-27-4, z.B. Mirapol® A-15)
- Polyquaternium-5: (Copolymeres aus dem Acrylamid und dem β-Methacryloxyethyltrimethylammoniummethosulfat, CAS-Nr. 26006-22-4)
- Polyquaternium-6: (Homopolymer des N,N-Dimethyl-N-2-propenyl-2-propen-1-aminiumchlorids, CAS-Nr. 26062-79-3, z.B. Merquat® 100
- Polyquaternium-7: N,N-Dimethyl-N-2-propenyl-2-propen-1-aminiumchlorid, Polymeres mit 2-Propenamid, CAS-Nr. 26590-05-6, z.B. Merquat® S
- Polyquaternium-10: Quaternäres Ammoniumsalz der Hydroxyethylcellulose, CAS-Nr. 53568-66-4, 55353-19-0, 54351-50-7, 68610-92-4, 81859-24-7, z.B. Celquat® SC-230M,
- Polyquaternium-11: Vinylpyrrolidon/dimethylaminoethyl-Methacrylat-Copolymer/Diethylsulfat-Reaktionsprodukt, CAS-Nr. 53633-54-8, z.B. Gafquat® 755N
- Polyquaternium-16: Vinylpyrrolidon/vinylimidazoliniummethochlorid-Copolymer, CAS-Nr. 29297-55-0, z.B. Luviquat® HM 552
- Polyquaternium-17: CAS-Nr. 90624-75-2, z.B. Mirapol® AD-1
- Polyquaternium-19: Quaternisierter wasserlöslicher Polyvinylalkohol
- Polyquaternium-20: in Wasser dispergierbarer quaternisierter Polyvinyloctadecylether
- Polyquaternium-21: Polysiloxan-polydimethyl-dimethylammoniumacetat-Copolymeres, z.B. Abil® B 9905
- Polyquaternium-22: Dimethyldiallylammoniumchlorid/Acrylsäure-Copolymer, CAS-Nr. 53694-7-0, z.B. Merquat® 280
- Polyquaternium-24: Polymeres quaternäres Ammoniumsalz der Hydroxyethylcellulose, Reaktionsprodukt mit einem mit Lauryldimethylammonium substituierten Epoxid, CAS-Nr. 107987-23-5, z.B. Quatrisoft® LM-200
- Polyquaternium-28: Vinylpyrrolidon/Methacrylamidopropyltrimethylammoniumchlorid-Copolymer, z.B. Gafquat® HS-100
- Polyquaternium-29: z.B. Lexquat® CH
- Polyquaternium-31: CAS-Nr. 136505-02-7, z.B. Hypan® QT 100
- Polyquaternium-32: N,N,N-trimethyl-2-[(2-methyl-1-oxo-2-propenyl)oxy]-Ethanaminiumchlorid, polymer mit 2-Propenamid, CAS-Nr. 35429-19-7
- Polyquaternium-37: CAS-Nr. 26161-33-1

Vorteilhaft enthalten erfindungsgemäße Zubereitungen zur Haarpflege 0.2 - 50 Gew.-% eines oder mehrerer Filmbildner, bevorzugt 5 - 30 Gew.-%, insbesondere 10 - 25 Gew.-%, jeweils bezogen auf das Gesamtgewicht der Zubereitungen. Derartige Ausführungsformen der erfindungsgemäßen Zubereitungen pflegen durch Umwelteinflüsse geschädigtes oder strapaziertes Haar bzw. beugen solchen Umwelteinflüssen vor. Ferner verleihen die erfindungsgemäßen Zubereitungen der Haartracht lockere Fülle und Festigkeit, ohne klebrig zu wirken.

Es ist gegebenenfalls möglich und vorteilhaft, die erfindungsgemäßen Zubereitungen als Grundlage für pharmazeutische Formulierungen zu verwenden. Mutatis mutandis gelten entsprechende Anforderungen an die Formulierung medizinischer Zubereitungen. Die Übergänge zwischen reinen Kosmetika und reinen Pharmaka sind dabei fließend. Als pharmazeutische Wirkstoffe sind erfindungsgemäß grundsätzlich alle Wirkstoffklassen geeginet, wobei lipophile Wirkstoffe bevorzugt sind. Beispiele sind: Antihistaminika, Antiphlogistika, Antibiotika, Antimykotika, die Durchblutung fördernde Wirkstoffe, Keratolytika, Hormone, Steroide, Vitamine usw.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Viruzide, Parfüme, Substanzen zum Verhindern des Schäumens, Farbstoffe, Pigmente, die färbende Wirkung haben, weitere, nicht unter die Definition der erfindungsgemäßen Verdicker fallende Verdickungsmittel, oberflächenaktive Substanzen, Emulgatoren, weichmachende, anfeuchtende und/oder feuchthaltende Substanzen, entzündungshemmende Substanzen, Medikamente, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel.

Insbesondere vorteilhaft werden Gemische der vorstehend genannten Lösungsmittel verwendet.

Als weitere Bestandteile können verwendet werden Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren, Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl,-monoethyl- oder -monobutylether, Diethylenglykolmonomethyl- oder -monoethylether und analoge Produkte.

Die erfindungsgemäßen Mikroemulsionsgele werden z.B. nach folgenden Methoden hergestellt:
a) Die Ölphase wird bei RT mit dem Emulgator oder der Emulgatorkombination versetzt und gerührt. Es ist vorteilhaft, die Mischung zu erwärmen, wenn feste Inhaltsstoffe zugesetzt werden. Zu dieser auf RT befindlichen Mikroemulsion wird die auf RT befindliche Wasserphase portionsweise unter Rühren zugesetzt. Zu dieser Mikroemulsion gibt man den erfindungsgemäßen Verdicker unter Rühren und erhält ein Gel. Löst sich der Verdicker schlecht, kann er auch von vornherein zur Ölphase gegeben werden.
b) Die Ölphase wird mit dem erfindungsgemäßen Emulgator oder der Emulgatorkombination versetzt und erhitzt in einem Bereich von z.B. 40 - 85 °C. Zu dieser Mischung fügt man portionsweise die 40 - 85 °C heiße Wasserphase und kühlt ab auf RT. Der Verdicker kann zu jedem Zeitpunkt der Herstellung zugefügt werden.
c) Der Emulgator/die Emulgatorkombination wird nur mit einem Teil der Ölphase versetzt. Anschließend wird Wasser portionsweise zugefügt und die erhaltene W/O-Mikroemulsion beziehungsweise das erhältliche Mikroemulsionsgel wird anschließend mit der restlichen Ölphase verdünnt. Dabei können intermediär weitere kolloidchemische Phasen (lamellare Phasen, hexagonale Phasen , invers hexagonale Phasen, kubische Phasen etc.) durchlaufen werden.
   Alle Mengenangaben, Prozentangaben oder Teile beziehen sich, soweit nicht anders angegeben, auf das Gewicht, insbesondere auf das Gesamtgewicht der Zubereitungen oder der jeweiligen Mischung.

Die nachfolgenden Beispiele sollen die vorliegende Erfindung verdeutlichen. Herstellvorschrift für die Vemetzer der Gruppe der hydrophil substituierten Polyisoprene:
Pl = Polyisopren
PEG = Polyethylenglycol
PS = Polystyrol

Die Zahlenangaben beziehen sich auf das Molekulargewicht.
a)
   Polyisopren mit Ethylenoxidendgruppen ( PI-50000 (PEG-5000)₂):
   Isopren wurde über Calciumchlorid getrocknet über 12 Stunden und anschließend desttilliert unter Argon. Direkt vor der Polymerisation wird wird eine Reinigung über Phenylmagnesiumchlorid angeschlossen. Das gereinigte Monomer wird in eine Ampulle über Argon aufbewahrt. Ethylenoxid wird auf gleiche Weise gereinigt. Die Polymerisation wird in einem Glasreaktor bei unter Argon durchgeführt. Es wird getrocknetes Tetrahydrofuran als Lösungsmittel benutzt. Die Polymerisation wird mit Kaliumnaphthaleid bei -40°C initiert. Nach 3 h Stunden wird Ethylenoxid zugefügt. Nach zwei Stunden wird die Temperatur auf 60°C erhöht und es wird über Nacht gerührt. Die Reaktion wird mit Methacryloylchlorid abgebrochen. Das Polymer wird durch wiederholte Fällung aus Methanol gereinigt und anschließend unter Vakuum getrocket. Die durch Lichtstreuung und Gelpermeationschromatographie ermittelten Molekulargewichte des ABA-Triblockpolymers ergeben folgende Werte:
   Polyisopropen: 50000 g/mol
   Polyethylenoxid: 5000 g/mol.
b)
   Ferner wurden weitere ABA-Triblockcopolymere durch Variation des Initiator/Monomer-Verhältnisses nach dieser Vorschrift hergestellt.
   (PI-50000 (PEG-10000)₂):
   Polyisopropen: 50000 g/mol
   Polyethylenoxid: 10000 g/mol.
c)
   Ferner wurden weitere ABA-Triblockcopolymere durch Variation des Initiator/Monomer-Verhältnisses nach dieser Vorschrift hergestellt.
   (PI-50000 (PEG-20000)₂):
   Polyisopropen: 50000 g/mol
   Polyethylenoxid: 20000 g/mol.
d)
   Ferner wurden weitere ABA-Triblockcopolymere durch Variation des Initiator/Monomer-Verhältnisses nach dieser Vorschrift hergestellt.
   PI-39000 (PEG-10000)₂
   Polyisopropen: 39000 g/mol
   Polyethylenoxid: 10000 g/mol.

Polyisopren mit ionischer Endgruppe (SO₃-):
Die Herstellung erfolgt analog zur o.a. Vorschrift bis zur Erzeugung des Polyisopren-Anions. Die anionischen Enden werden äquimolar mit 1,1 Diphenylethan (über Natrium destilliert) umgesetzt und die Lösung wird auf 168 K (Tetrahydrofuran/ fl. Stickstoff )abgekühlt und ein Überschuß an 1,3 Propansultan zugegeben. Die Lösung wird langsam auf Raumperatur gebracht. Die Aufarbeitung erfolgt analog zur der des bis-Polyethylenoxid modifizierten Polyisoprens.
Die Variation des Gegenions kann durch Verwendung von Kalium oder Lithium bei der Startreaktion erreicht werden.
Pl (SO₃Li)₂ (20000 g/mol)
Pl (SO₃Na)₂ (50000 g/mol),

Polyisopren mit ionischer Endgruppe Carboxylat:
Man verwendet an Stelle des 1,3 Propansultons in der o.a. Vorschrift das Gammabutyrolacton.
PI(CO₂Na)₂ (65000 g/mol)

Die folgenden Zahlenangaben sind Gew.-%.
Die Beispiele 1 bis 11 betreffen erfindungsgemäße niedrigviskose Mikroemulsionen, die Beispiele 12 bis 22 beinhalten die erfindungsgemäßen Mikoemulsionsgele.

Das verwendete Dimethicon Copolyol ist in der Beschreibung mit A1 eindeutig als Verdicker bzw. Vernetzer definiert.

### Beispiel 1:

W/O-Mikroemulsion, niedrigviskos (Pumpspray, Deospray-Konzentrat)

| | |
|---|---|
| PEG-5 Soya Sterol | 4.70 |
| Natrium Lauroyl Lactylat | 2.30 |
| Dicaprylylether | 60.0 |
| Wasser | 33.0 |

### Beispiel 2:

W/O-Mikroemulsion, niedrigviskos (Antiakne Lotion)

| | |
|---|---|
| PEG-5 Soya Sterol | 4.70 |
| Natrium Lauroyl Lactylat | 2.30 |
| Dicaprylylether | 60.0 |
| Wasser | **33.0** |

### Beispiel 3:

W/O-Mikroemulsion, niedrigviskos (desodorierende Körperlotion)

| | |
|---|---|
| Glycerylisostearat | 5.00 |
| Natrium Lauroyl Lactylat | 2.00 |
| Dicaprylylether | 60.0 |
| Wasser | 33.0 |

### Beispiel 4:

W/O-Mikroemulsion, niedrigviskos (Aftershave-Lotion)

| | |
|---|---|
| 2-Ethylhexylglycerinether | 6.00 |
| Natrium Lauroyl Lactylat | 1.00 |
| Dicaprylylether | 60.0 |
| Wasser | 33.0 |

### Beispiel 5:

W/O-Mikroemulsion, niedrigviskos (Augenmakeup-Entferner Lotion)

| | |
|---|---|
| PEG-5 Soya Sterol | 3.50 |
| Natrium Caproyl Lactylat | 3.50 |
| Dicaprylylether | 60.0 |
| Wasser | 33.0 |

### Beispiel 6:

W/O-Mikroemulsion, niedrigviskos (Rasierlotion)

| | |
|---|---|
| Natrium Lauroyl Lactylat | 5.00 |
| Glyceryl Caprylat | 2.00 |
| Dicaprylylether | 60.0 |
| Wasser | 33.0 |

### Beispiel 7:

W/O-Mikroemulsion, niedrigviskos (Lotion für trockene Haut)

| | |
|---|---|
| Natrium Lauroyl Lactylat | 5.00 |
| Glyceryl Caprylat | 2.00 |
| Dicaprylylether | 60.0 |
| Wasser | 33.0 |

### Beispiel 8:

W/O-Mikroemulsion, niedrigviskos (Duschlotion)

| | |
|---|---|
| 2-Ethylhexylglycerinether | 4.43 |
| Natrium laureth sulfat | 1.77 |
| Dicaprylylether | 53.09 |
| Wasser | 40.71 |

### Beispiel 9

W/O-Mikroemulsion, niedrigviskos (Grundlage für Antitranspirant-Lotion, Enzyme, Proteine)

| | |
|---|---|
| Polyglycerinmonoisostearat (Polydermanol GE 14 DA) | 5.00 |
| Polyglycerindisostearat (Polydermanol GE 60 DS) | 2.00 |
| Isopropylmyristat | 60.0 |
| Wasser | 32.0 |

### Beispiel 10:

| | |
|---|---|
| Polyglycerin Triisostearat | 5.88 |
| (W/O-Mikroemulsion, niedrigviskos (Grundlage für Vitamin C, wasserlösliche UV-Filter) Polydermanol GE 40 DS) PEG(15)Cetylether | 2.35 |
| Isopropylmyristat , | 70.59 |
| Wasser | 21.18 |

### Beispiel 11

W/O-Mikroemulsion, niedrigviskos (Haarlotion, Haarspray-Konzentrat)

| | |
|---|---|
| Cetyltrimethylamoniumbromid | 1.10 |
| 2-Ethylhexylglycerinether | 6.90 |
| Dioctylcyclohexan | 69.0 |
| Wasser | **23.0** |

### Beispiel 12:

W/O-Mikroemulsion, Gel (Deogel)

| | |
|---|---|
| PEG-5 Soya Sterol | 4.70 |
| Natrium Lauroyl Lactylat | 2.30 |
| Dicaprylylether | 52.02 |
| Wasser | 33.0 |
| PI-50000 (PEG-5000)2 | 7.98 |

### Beispiel 13:

W/O-Mikroemulsion, Gel (Anti-Akne-Gel, Rasiergel-Konzentrat)

| | |
|---|---|
| PEG-5 Soya Sterol | 4.70 |
| Natrium Lauroyl Lactylat | 2.30 |
| Dicaprylylether | 53.0 |
| Wasser | 33.0 |
| Pl (SO₃Li)₂ (20000 g/mol) | 7.00 |

### Beispiel 14:

W/O-Mikroemulsion, Gel (Rasierschaum-Grundlage)

| | |
|---|---|
| Glycerylisostearat | 5.00 |
| Natrium Lauroyl Lactylat | 2.00 |
| Dicaprylylether | 52.9 |
| Wasser | 33.0 |
| PI(CO₂Na)₂ (65000 g/mol) | 7.10 |

### Beispiel 15:

W/O-Mikroemulsion, Gel (Aftershave-Gel)

| | |
|---|---|
| 2-Ethylhexylglycerinether | 6.00 |
| Natrium Lauroyl Lactylat | 1.00 |
| Dicaprylylether | 52.60 |
| Wasser | 33.0 |
| PI-39000 (PEG-10000)₂ | 7.40 |

### Beispiel 16:

W/O-Mikroemulsion, Gel (Augenmakeup-Enffernergel)

| | |
|---|---|
| PEG-5 Soya Sterol | 3.50 |
| Natrium Caproyl Lactylat | 3.50 |
| Dicaprylylether | 52.72 |
| Wasser | 33.0 |
| PI-50000 (PEG-10000)₂ | 7.28 |

### Beispiel 17:

W/O-Mikroemulsion, Gel (Hautpflegegel)

| | |
|---|---|
| Natrium Lauroyl Lactylat | 5.00 |
| Glyceryl Caprylat . | 2.00 |
| Dicaprylylether | 52.72 |
| Wasser | 33.0 |
| PI-50000 (PEG-20000)₂ | 7.28 |

### Beispiel 18:

W/O-Mikroemulsion, Gel (Gel für trockene Haut)

| | |
|---|---|
| Natrium Lauroyl Lactylat | 5.00 |
| Glyceryl Caprylat | 2.00 |
| Dicaprylylether | 53.9 |
| Wasser | 33.0 |
| PI (SO₃Li)₂ (20000 g/mol) | 6.40 |

### Beispiel 19:

W/O-Mikroemulsion, Gel (Duschgel)

| | |
|---|---|
| 2-Ethylhexylglycerinether | *4.43* |
| Natrium laureth sulfat | 1.77 |
| Dicaprylylether | 45.59 |
| PI-50000 (PEG-5000)₂ | 7.50 |
| Wasser | 40.71 |

### Beispiel 20:

W/O-Mikroemulsion, Gel (Antitranspirant-Grundlage, Gel zur Solubilisierung von Enzymen, Proteinen))

| | |
|---|---|
| Polyglycerinmonoisostearat (Polydermanol GE 14 DA) | 5.00 |
| Polyglycerindisostearat (Polydermanol GE 60 DS) | 2.00 |
| Isopropylmyristat | 60.0 |
| PI-50000 (PEG-10000)₂ | 7.50 |
| Wasser | 32.0 |

### Beispiel 21:

W/O-Mikroemulsion, Gel (Grundlage für Vitamin C, wasserlösliche UV-Filter)

| | |
|---|---|
| Polyglycerin Triisostearat (Polydermanol GE 40 DS) | 5.88 |
| PEG(15) Cetylether | 2.35 |
| Isopropylmyristat Dimethicon Copolyol A1 | 63.09 |
| (Goldschmidt) | 7.50 |
| Wasser | 21.18 |

### Beispiel 22:

W/O-Mikroemulsion, Gel (Haargel)

| | |
|---|---|
| Cetyltrimethylamoniumbromid | 1.10 |
| 2-Ethylhexylglycerinether | 6.90 |
| Dioctylcyclohexan | 62.00 |
| PI-50000 (PEG-10000)₂ | 7.00 |
| Wasser | 23.0 |

W/O Mikroemulsionen ohne Verdicker (Beispiele 23 - 29):

### Beispiel 23:

| | |
|---|---|
| PEG-20 Sorbitan Monolaurate | 2.15 |
| Polyglycerin Triisostearate (Polydermanol GE 40 DS) | 5.38 |
| Isopropyl Myristate | 64.47 |
| Wasser | 28 |

### Beispiel 24:

| | |
|---|---|
| Polyglycerin Diisostearate | 7.45 |
| Isopropyl Myristate | 63.82 |
| Wasser | 28.73 |

### Beispiel 25:

| | |
|---|---|
| Steareth-2 | 5.66 |
| Steareth-16 | 2.77 |
| Cetearyl Isononanoate | 72.29 |
| Wasser | 19.28 |

### Beispiel 26:

| | |
|---|---|
| PEG-20 Octyldodecylether | 4.12 |
| Ethylhexylglycerinether | 4.12 |
| Dicaprylylether | 70.58 |
| Wasser | 21.18 |

### Beispiel 27:

| | |
|---|---|
| Sorbitanlaurate | 3.53 |
| PEG-20 Sorbitantrioleate | 4.71 |
| Dicaprylylether | 70.58 |
| Wasser | 21.18 |

### Beispiel 28:

| | |
|---|---|
| Glyceryl Isostearate | 3.0 |
| PEG-12 Distearate | 4.0 |
| Dicaprylylether | 60.0 |
| Wasser | 33.0 |

### Beispiel 29:

| | |
|---|---|
| Lecithin | 6.71 |
| Polyglyceryl-2 Caprate | 1.83 |
| Caprylic/Capric Triglyceride | 68.16 |
| Wasser | 17.20 |
| Propylenglycol | 6.1 |

W/O Mikroemulsionen mit Verdicker, Gele

### Beispiel 30:

| | |
|---|---|
| PEG-20 Sorbitan Monolaurate | 2.15 |
| Polyglycerin Triisostearate (Polydermanol GE 40 DS) | 5.38 |
| Isopropyl Myristate | 56.97 |
| Wasser | 28 |
| PI-50000(PEG-10000)₂ | 7.5 |

### Beispiel 31:

| | |
|---|---|
| Polyglycerin Diisostearate | 7.45 |
| Isopropyl Myristate | 55.82 |
| Wasser | 28.73 |
| PI-50000(PEG-10000)₂ | 8 |

### Beispiel 32:

| | |
|---|---|
| Steareth-2 | 5.66 |
| Steareth-16 | 2.77 |
| Cetearyl Isononanoate | 65.29 |
| Wasser | 19.28 |
| PI-50000(PEG-10000)₂ | 7 |

### Beispiel 33:

| | |
|---|---|
| PEG-20 Octyldodecylether | 4.12 |
| Ethylhexylglycerinether | 4.12 |
| Dicaprylylether | 63.28 |
| Wasser | 21.18 |
| PI-50000(PEG-10000)₂ | 7.3 |

### Beispiel 34:

| | |
|---|---|
| Sorbitanlaurate | 3.53 |
| PEG-20 Sorbitantrioleate | 4.71 |
| Dicaprylylether | 63.58 |
| Wasser | 21.18 |
| PI-50000(PEG-10000)₂ | 7 |

### Beispiel 35:

| | |
|---|---|
| Glyceryl Isostearate | 3.0 |
| PEG-12 Distearate | 4.0 |
| Dicaprylylether | 53.0 |
| Wasser | 33.0 |
| PI-50000(PEG-10000)₂ | 7 |

### Beispiel 36:

| | |
|---|---|
| Lecithin | 6.71 |
| Polyglyceryl-2 Caprate | 1.83 |
| Caprylic/Capric Triglyceride | 61.16 |
| Wasser | 17.20 |
| PI-50000(PEG-10000)₂ | 7 |
| Propylenglycol | 6.1 |

## Patentansprüche

1. Mikroemulsionsgele,
(a) beruhend auf Mikroemulsionen vom Typ Wasser in Öl, welche umfassen
- eine Ölphase, welche im wesentlichen aus schwerflüchtigen Bestandteilen zusammengesetzt ist, und eine Wasserphase
- enthaltend:
einen oder mehrere polyethoxylierte W/O-Emulgatoren und/oder
einen oder mehrere polypropoxylierte W/O-Emulgatoren und/oder
einen oder mehrere polyethoxylierte und polypropoxylierte W/O-Emulgatoren und/oder
- einen oder mehrere Monoester, Diester, Polyester von Polyolen als W/O-Emulgatoren und/oder
- einen oder mehrere Monoether, Diether, Polyether von Polyolen als W/O-Emulgatoren und/oder
- einen oder mehrere Dimethicon copolyole als W/O Emulgatoren und/oder
- einen oder mehrere Fettalkohole oder Fettsäuren als W/O-Emulgatoren und/oder
- einen oder mehrere Sorbitanester als W/O-Emulgatoren und/oder
- einen oder mehrere Methylglucoseester als W/O-Emulgatoren
- gewünschtenfalls ferner enthaltend einen oder mehrere O/W-Emulgatoren
- erhältlich auf die Weise, daß ein Gemisch aus den Grundkomponenten, umfassend Wasserphase, Ölphase, einen oder mehrere der erfindungsgemäßen Emulgatoren hergestellt wird, wobei der HLB-Wert des Emulgators oder der Emulgatorkombination im Bereich von 2 - 14 liegt,
(b) bei welcher die Tröpfchen der diskontinuierlichen Wasserphase durch eine oder mehrere Vemetzersubstanzen miteinander verbunden sind, deren Moleküle sich durch mindestens einen hydrophoben Bereich auszeichnen, welcher eine Ausdehnung aufweist, die geeignet ist, den Abstand der Mikroemulsionströpfchen untereinander zu überbrücken, und durch mindestens einen hydrophilen Bereich, welcher mit den Mikroemulsionströpfchen in hydrophile Wechselwirkung zu treten vermag.

2. Mikroemulsionsgele,
(a) beruhend auf Mikroemulsionen vom Typ Wasser-in-Öl, welche umfassen
- eine kontiniuerliche Ölphase und eine diskontinuierliche Wasserphase
- enthaltend mindestens einen
einen oder mehrere polyethoxylierte W/O-Emulgatoren und/oder
einen oder mehrere polypropoxylierte W/O-Emulgatoren und/oder
einen oder mehrere polyethoxylierte und polypropoxylierte W/O-Emulgatoren und/oder
einen oder mehrere Monoester, Diester, Polyester von Polyolen als W/O-Emulgatoren und/oder
- einen oder mehrere Monoether von Polyolen und deren Ester als W/O-Emulgatoren und/oder
- einen oder mehrere Sorbitanester als W/O-Emulgatoren
- einen oder mehrere Silikonemulgatoren als W/O Emulgatoren und/oder
- einen oder mehrere Fettalkohole oder Fettsäuren als W/O-Emulgatoren
- wobei dieser W/O-Emulgator ausgewählt wird aus der Gruppe der
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 1 bis 10 darstellen
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 1 bis 30 darstellen
- der Fettsäureethoxylate der allgemeinen Formel R-COO-(-CH₂-CH₂-O-)ₙ-H, wobei R einen verzweigte oder unverzweigten Alkyloder Alkenylreste und n eine Zahl von 1 bis 20 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste und n eine Zahl von 1 bis 20 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl, Hydroxyalkyl - oder Alkenylreste und n eine Zahl von 1 bis 40 darstellen,
- der veretherten Fettsäureethoxylate der allgemeinen Formel R-COO-(-CH₂-CH₂-O-)ₙ-R', wobei R und R' unabhängig voneinander verzweigte oder unverzweigte Alkyl- oder Alkenylreste und n eine Zahl von 1 bis 40 darstellen
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 1 bis 30 darstellen,
- der Polyoxyethylensorbitanfettsäureester, basierend auf verzweigten oder unverzweigten Alkan- oder Alkensäuren und einen Ethoxylierungsgrad von 1 bis 10 aufweisend
- der Cholesterinethoxylate mit einem Ethoxylierungsgrad zwischen 1 und 10,
- der ethoxlierten Glyceride mit einem Ethoxylierungsgrad von 1 bis 30
- der ethoxylierten Triglyceride mit einem Ethoxylierungsgrad zwischen 1 und 30,
- der Monoglycerinether des Typs R-O-CH2-C(H)OH-CH2OH wobei R einen verzweigten oder unverzweigten Alkyl-, Aryl- oder Alkenylrest darstellen und
- der Monoglycerinester des Typs RC(O)OCH2-C(H)OH-CH2OH wobei R einen verzweigten oder unverzweigten Alkyl-, Hydroxyalkyl, Aryl- oder Alkenylrest darstellen
- der Diglycerinester des Typs RC(O)OCH2-C(H)OH-CH2OC(O)R', wobei wobei R und R' unabhängig voneinander verzweigte oder unverzweigte Alkyl- , Hydroxyalkyl oder Alkenylreste und n eine Zahl von 1 bis 30 oder darstellen,
- der Polyglycerinmono- oder di- oder polyester, wobei die Fettsäuren unabhängig voneinander verzweigte oder unverzweigte Alkyl- , Hydroxyalkyl oder Alkenylreste darstellen,
- der Pentaerythritester wobei die Fettsäuren unabhängig voneinander verzweigte oder unverzweigte Afkyl-, Hydroxyalkyl oder Alkenylreste dar-stellen,
- der Propylenglycolester, wobei die Fettsäuren unabhängig voneinander verzweigte oder unverzweigte Alkyl-, Hydroxyalkyl oder Alkenylreste dar-stellen,
- der Sorbitanester, wobei die Fettsäuren unabhängig voneinander verzweigte oder unverzweigte Alkyl-, Hydroxyalkyl oder Alkenylreste darstellen,
- der Fettalkohole R-OH und Fettsäuren RCOOH, wobei R einen verzweigten oder unverzweigten Alkyl- oder Alkenylrest darstellen,
- der Silikonemulgatoren
- der Methylglucoseester, wobei die Fettsäuren unabhängig voneinander verzweigte oder unverzweigte Alkyl-, Hydroxyalkyl oder Alkenylreste darstellen,
- gewünschtenfalls enthaltend einen oder mehrere O/W-Emulgatoren
- einen Gesamtemulgatorgehalt vorzugsweise kleiner als 20 Gew.-%, bezogen auf das Gesamtgewicht der Emulsion, aufweisend,
- erhältlich auf die Weise, daß ein Gemisch aus den Grundkomponenten, umfassend Wasserphase, Ölphase, einen oder mehrere der erfindungsgemäßen W/O-Emulgatoren, gewünschtenfalls einen oder mehrere O/W-Emulgatoren hergestellt wird, wobei der HLB-Wert des Emulgators oder der Emulgatorkombination im Bereich von 2 - 14 liegt,
(b) bei welcher die Tröpfchen der diskontinuierlichen Wasserphase durch eine oder mehrere Vernetzersubstanzen miteinander verbunden sind, deren Moleküle sich durch mindestens einen hydrophoben Bereich auszeichnen, welcher eine Ausdehnung aufweist, die geeignet ist, den Abstand der Mikroemulsionströpfchen untereinander zu überbrücken, und durch mindestens einen hydrophilen Bereich, welcher mit den Mikroemulsionströpfchen in hydrophile Wechselwirkung zu treten vermag.

## Claims

1. Microemulsion gels
(a) based on microemulsions of the water-in-oil type, which comprise
- an oily phase, which is essentially composed of constituents of low volatility, and an aqueous phase
- comprising:
one or more polyethoxylated W/O emulsifiers and/or
one or more polypropoxylated W/O emulsifiers and/or
one or more polyethoxylated and polypropoxylated W/O emulsifiers, and/or
- one or more monoesters, diesters, polyesters of polyols as W/O emulsifiers and/or
- one or more monoethers, diethers, polyethers of polyols as W/O emulsifiers and/or
- one or more dimethicone copolyols as W/O emulsifiers and/or
- one or more fatty alcohols or fatty acids and W/O emulsifiers and/or
- one or more sorbitan esters as W/O emulsifiers and/or
- one or more methylglucose esters as W/O emulsifiers
- if desired furthermore comprising one or more O/W emulsifiers
- obtainable by preparing a mixture of the base components, comprising the aqueous phase, the oily phase and one or more of the emulsifiers according to the invention, the HLB value of the emulsifier or of the emulsifier combination being in the range 2-14,
(b) in which the droplets of the discontinuous aqueous phase are joined to one another by one or more crosslinking substances, the molecules of which are distinguished by at least one hydrophobic region, which has an extension which is capable of bridging the distance between the microemulsion droplets, and by at least one hydrophilic range, which is capable of entering into a hydrophilic interaction with the microemulsion droplets.

2. Microemulsion gels
(a) based on microemulsions of the water-in-oil type, which comprise
- a continuous oily phase and a discontinuous aqueous phase
- comprising at least
one or more polyethoxylated W/O emulsifiers and/or
one or more polypropoxylated W/O emulsifiers and/or
one or more polyethoxylated and polypropoxylated W/O emulsifiers and/or
one or more monoesters, diesters, polyesters of polyols as W/O emulsifiers and/or
- one or more monoethers of polyols and esters thereof as W/O emulsifiers and/or
- one or more sorbitan esters as W/O emulsifiers
- one or more silicone emulsifiers as W/O emulsifiers and/or
- one or more fatty alcohols or fatty acids as W/O emulsifiers
- where this W/O emulsifier is chosen from the group consisting of
- fatty alcohol ethoxylates of the general formula R-O-(-CH₂-CH₂-O-)ₙ-H, where R is a branched or unbranched alkyl, aryl or alkenyl radical, and n is a number from 1 to 10,
- polyethylene glycol ethers of the general formula R-O-(-CH₂-CH₂-O-)ₙ-R', where R and R' independently of one another are branched or unbranched alkyl or alkenyl radicals, and n is a number from 1 to 30,
- fatty acid ethoxylates of the general formula R-COO-(-CH₂-CH₂-O-)ₙ-H, where R is a branched or unbranched alkyl or alkenyl radical, and n is a number from 1 to 20,
- esterified fatty acid ethoxylates of the general formula R-COO- (-CH₂-CH₂-O-)ₙ-C(O)-R' where R and R' independently of one another are branched or unbranched alkyl or alkenyl radicals, and n is a number from 1 to 20,
- esterified fatty acid ethoxylates of the general formula R-COO-(-CH₂-CH₂-O-)ₙ-C(O)-R', where R and R' independently of one another are branched or unbranched alkyl, hydroxyalkyl or alkenyl radicals, and n is a number from 1 to 40,
- etherified fatty acid ethoxylates of the general formula R-COO-(-CH₂-CH₂-O-)ₙ-R', where R and R' independently of one another are branched or unbranched alkyl or alkenyl radicals, and n is a number from 1 to 40
- fatty alcohol propoxylates of the general formula R-O-(-CH₂-CH(CH₃)-O-)ₙ-H, where R is a branched or unbranched alkyl or alkenyl radical, and n is a number from 1 to 30,
- polyoxyethylene sorbitan fatty acid esters based on branched or unbranched alkanoic or alkenoic acids and having a degree of ethoxylation of from 1 to 10,
- cholesterol ethoxylates having a degree of ethoxylation between 1 and 10,
- ethoxylated glycerides having a degree of ethoxylation of from 1 to 30,
- ethoxylated triglycerides having a degree of ethoxylation between 1 and 30,
- monoglycerol ethers of the type R-O-CH₂-C(H)OH-CH₂OH, where R is a branched or unbranched alkyl, aryl or alkenyl radical, and
- monoglycerol esters of the type RC(O)OCH₂-C(H)OH-CH₂OH where R is a branched or unbranched alkyl, hydroxyalkyl, aryl or alkenyl radical,
- diglycerol esters of the type RC(O)OCH₂-C(H)OH-CH₂OC(O)R', where R and R' independently of one another are branched or unbranched alkyl, hydroxyalkyl or alkenyl radicals, and n is a number from 1 to 30,
- polyglycerol mono- or di- or polyesters, where the fatty acids independently of one another are branched or unbranched alkyl, hydroxyalkyl or alkenyl radicals,
- pentaerythritol esters, where the fatty acids independently of one another are branched or unbranched alkyl, hydroxyalkyl or alkenyl radicals,
- propylene glycol esters, where the fatty acids independently of one another are branched or unbranched alkyl, hydroxyalkyl or alkenyl radicals,
- sorbitan esters, where the fatty acids independently of one another are branched or unbranched alkyl, hydroxyalkyl or alkenyl radicals,
- fatty alcohols R-OH and fatty acids RCOOH, where R is a branched or unbranched alkyl or alkenyl radical,
- silicone emulsifiers
- methylglucose esters, where the fatty acids independently of one another are branched or unbranched alkyl, hydroxyalkyl or alkenyl radicals,
- if desired comprising one or more O/W emulsifiers
- having a total emulsifier content of preferably less than 20% by weight, based on the total weight of the emulsion,
- obtainable by preparing a mixture of the base components, comprising aqueous phase, oily phase, one or more of the W/O emulsifiers according to the invention, if desired one or more O/W emulsifiers, where the HLB value of the emulsifier or of the emulsifier combination is in the range 2-14,
(b) in which the droplets of the discontinuous aqueous phase are joined to one another by one or more crosslinking substances, the molecules of which are distinguished by at least one hydrophobic region which has an extension which is capable of bridging the distance between the microemulsion droplets, and by at least one hydrophilic region which is capable of entering into hydrophilic interaction with the microemulsion droplets.

## Revendications

1. Gels en microémulsion,
(a) à base de microémulsions du type eau/huile, comprenant
- une phase huileuse qui est essentiellement composée de constituants de faible volatilité, et une phase aqueuse
- comprenant:
un ou plusieurs émulsifiants eau/huile polyéthoxylés et/ou
un ou plusieurs émulsifiants eau/huile polypropoxylés et/ou
un ou plusieurs émulsifiants eau/huile polyéthoxylés et
polypropoxylés et/ou
- un ou plusieurs monoesters, diesters, polyesters de polyols en tant qu'émulsifiants eau/huile et/ou
- un ou plusieurs monoéthers, diéthers, polyéthers de polyols en tant qu'émulsifiants eau/huile et/ou
- un ou plusieurs diméthicone-copolyols en tant qu'émulsifiants eau/huile et/ou
- un ou plusieurs alcools gras ou acides gras en tant qu'émulsifiants eau/huile et/ou
- un ou plusieurs esters de sorbitane en tant qu'émulsifiants eau/huile et/ou
- un ou plusieurs esters méthylglucosiques en tant qu'émulsifiants eau/huile
- comprenant en outre, si on le souhaite, un ou plusieurs émulsifiants huile/eau
- pouvant être obtenus en préparant un mélange des composants de base, comprenant la phase aqueuse, la phase huileuse et un ou plusieurs des émulsifiants selon l'invention, la valeur de HLB de l'émulsifiant ou de la combinaison d'émulsifiants étant dans la gamme de 2 à 14,
(b) dans lesquels les gouttelettes de la phase aqueuse discontinue sont liées les unes aux autres par une ou plusieurs substances de réticulation dont les molécules se distinguent par au moins une région hydrophobe, présentant une extension qui est appropriée pour réaliser un pont sur la distance entre les gouttelettes en microémulsion les unes en dessous des autres, et par au moins une région hydrophile qui est capable d'entrer en interaction hydrophile avec les gouttelettes en microémulsion.

2. Gels en microémulsion,
(a) à base de microémulsions du type eau/huile, comprenant
- une phase huileuse continue et une phase aqueuse discontinue
- comprenant au moins
un ou plusieurs émulsifiants eau/huile polyéthoxylés et/ou
un ou plusieurs émulsifiants eau/huile polypropoxylés et/ou
un ou plusieurs émulsifiants eau/huile polyéthoxylés et polypropoxylés et/ou
un ou plusieurs monoesters, diesters, polyesters de polyols en tant qu'émulsifiants eau/huile et/ou
- un ou plusieurs monoéthers de polyols et leurs esters en tant qu'émulsifiants eau/huile et/ou
- un ou plusieurs esters de sorbitane en tant qu'émulsifiants eau/huile
- un ou plusieurs émulsifiants siliconés en tant qu'émulsifiants eau/huile et/ou
- un ou plusieurs alcools gras ou acides gras en tant qu'émulsifiants eau/huile
- cet émulsifiant eau/huile étant choisi parmi le groupe constitué
- des éthoxylates d'alcools gras de formule générale R-O-(CH₂-CH₂-O)ₙ-H, dans laquelle R représente un radical alkyle, aryle ou alcényle ramifié ou non ramifié et n représente un nombre valant de 1 à 10
- des éthers de polyéthylèneglycol de formule générale R-O-(CH₂-CH₂-O)ₙ-R', dans laquelle R et R' représentent, indépendamment l'un de l'autre, des radicaux alkyle ou alcényle ramifiés ou non ramifiés et n représente un nombre valant de 1 à 30
- des éthoxylates d'acides gras de formule générale R-COO-(CH₂-CH₂-O)ₙ-H, dans laquelle R représente un radical alkyle ou alcényle ramifié ou non ramifié et n représente un nombre valant de 1 à 20
- des éthoxylates d'acides gras estérifiés de formule générale R-COO-(CH₂-CH₂-O)ₙ-C(O)-R', dans laquelle R et R' représentent, indépendamment l'un de l'autre, des radicaux alkyle ou alcényle ramifiés ou non ramifiés et n représente un nombre valant de 1 à 20
- des éthoxylates d'acides gras estérifiés de formule générale R-COO-(CH₂-CH₂-O)ₙ-C(O)-R', dans laquelle R et R' représentent, indépendamment l'un de l'autre, des radicaux alkyle, hydroxyalkyle ou alcényle ramifiés ou non ramifiés et n représente un nombre valant de 1 à 40
- des éthoxylates d'acides gras ethérifiés de formule générale R-COO-(CH₂-CH₂-O)ₙ-R', dans laquelle R et R' représentent, indépendamment l'un de l'autre, des radicaux alkyle ou alcényle ramifiés ou non ramifiés et n représente un nombre valant de 1 à 40
- des propoxylates d'alcools gras de formule générale R-O-(CH₂-CH(CH₃)-O)ₙ-H, dans laquelle R représente un radical alkyle ou alcényle ramifié ou non ramifié et n représente un nombre valant de 1 à 30
- des esters d'acides gras et de polyoxyéthylènesorbitane à base d'acides alcanoïques ou alcénoïques ramifiés ou non ramifiés et présentant un degré d'éthoxylation de 1 à 10
- des éthoxylates de cholestérol présentant un degré d'éthoxylation compris entre 1 et 10
- des glycérides éthoxylés présentant un degré d'éthoxylation de 1 à 30
- des triglycérides éthoxylés présentant un degré d'éthoxylation compris entre 1 et 30
- des éthers de monoglycérol du type R-O-CH2-C(H)OH-CH20H, dans lesquels R représente un radical alkyle, aryle ou alcényle ramifié ou non ramifié, et
- des esters de monoglycérol du type RC(O)OCH2-C(H)OH-CH2OH, dans lesquels R représente un radical alkyle, hydroxyalkyle, aryle ou alcényle ramifié ou non ramifié
- des esters de diglycérol du type RC(O)OCH2-C(H)OH-CH2OC(O)R', dans laquelle R et R' représentent, indépendamment l'un de l'autre, des radicaux alkyle, hydroxyalkyle ou alcényle ramifiés ou non ramifiés et n représente un nombre valant de 1 à 30
- des monoesters, diesters ou polyesters de polyglycérol, dans lesquels les acides gras représentent, indépendamment les uns des autres, des radicaux alkyle, hydroxyalkyle ou alcényle ramifiés ou non ramifiés
- des esters de pentaérythritol, dans lesquels les acides gras représentent, indépendamment les uns des autres, des radicaux alkyle, hydroxyalkyle ou alcényle ramifiés ou non ramifiés
- des esters de propylèneglycol, dans lesquels les acides gras représentent, indépendamment les uns des autres, des radicaux alkyle, hydroxyalkyle ou alcényle ramifiés ou non ramifiés
- des esters de sorbitane, dans lesquels les acides gras représentent, indépendamment les uns des autres, des radicaux alkyle, hydroxyalkyle ou alcényle ramifiés ou non ramifiés
- des alcools gras R-OH et des acides gras RCOOH, dans lesquels R représente un radical alkyle ou alcényle ramifié ou non ramifié
- des émulsifiants siliconés
- des esters méthylglucosiques, dans lesquels les acides gras représentent, indépendamment les uns des autres, des radicaux alkyle, hydroxyalkyle ou alcényle ramifiés ou non ramifiés
- comprenant, si on le souhaite, un ou plusieurs émulsifiants huile/eau
- présentant une teneur totale en émulsifiants de préférence inférieure à 20 % en poids, par rapport au poids total de l'émulsion
- pouvant être obtenue en préparant un mélange des composants de base, comprenant la phase aqueuse, la phase huileuse et un ou plusieurs des émulsifiants eau/huile selon l'invention, si on le souhaite, un ou plusieurs émulsifiants huile/eau, la valeur de HLB de l'émulsifiant ou de la combinaison d'émulsifiants étant dans la gamme de 2 à 14,
(b) dans lesquels les gouttelettes de la phase aqueuse discontinue sont liées les unes aux autres par une ou plusieurs substances de réticulation dont les molécules se distinguent par au moins une région hydrophobe, présentant une extension qui est appropriée pour réaliser un pont sur la distance entre les gouttelettes en microémulsion les unes en dessous des autres, et par au moins une région hydrophile qui est capable d'entrer en interaction hydrophile avec les gouttelettes en microémulsion.
